# EUROPEAN PATENT SPECIFICATION

(11) **EP 4 368 110 B1**
(45) Date of publication and mention of the grant of the patent: **19.08.2026**
(21) Application number: 23204070.9
(22) Date of filing: 17.10.2023
(51) Int. Cl.: A61B 6/04, A61B 6/50

(54) **METHODS AND SYSTEMS FOR A CRADLE INTERFACE PAD**
VERFAHREN UND SYSTEME FÜR EIN WIEGENSCHNITTSTELLENPAD
PROCÉDÉS ET SYSTÈMES POUR UN PAVÉ D'INTERFACE DE BERCEAU

(30) Priority: 10.11.2022 US 202218054402
(43) Date of publication of application: 15.05.2024
(73) Proprietor: GE Precision Healthcare LLC, Waukesha, WI 53188 (US)
(72) Inventor: LEWIS, Chelsey Amanda, Waukesha, 53188 (US); DELONG SAMALIK, Michelle Marie Severino, Waukesha, 53188 (US); EVANGELIST, Matthew J., Waukesha, 53188 (US); SMITH, Chad A., Waukesha, 53188 (US); NETT, Brian E., Waukesha, 53188 (US)
(74) Representative: Kilburn & Strode LLP

(56) References cited:
- WO-A1-2017/039522
- JP-A- 2000 116 645
- JP-A- 2002 291 731
- US-A- 4 400 820
- US-A1- 2003 159 216
- US-A1- 2007 270 683
- US-A1- 2019 277 927

## Description

### TECHNICAL FIELD

Embodiments of the subject matter disclosed herein relate to methods and systems for supporting a head or extremity of a patient body.

### BACKGROUND

A support system for a patient head and/or extremity may be used in a variety of environments. Support systems capable of providing a range of angles at which a patient head and/or extremity may be positioned may be used during imaging scans to support and position a patient head and/or limb through a range of discrete angles. The ability to tilt a patient's head and/or limb and hold that position throughout an imaging scan is may affect image quality. In one example, support systems for a head may be used in a medical setting to select and maintain the position of a patient's head to avoid imaging dental implants and other devices that may create image artifacts during a medical imaging procedure. In addition, tilting a patient's head may allow a reduction in radiation dose to a sensitive anatomy, such as the eyes, by placing them outside of the x-ray radiation beam. A support system for a patient's head and/or extremity allows an imaging technologist or operator to position a patient's head and/or extremity in such a way that without the device, the patient may not be able to hold their head and/or extremity in a specific orientation during a scan. Other patients who would benefit from the use of a head and/or extremity support system are patients who can be subject to involuntary movements or may be combative. Additionally, in emergency situations where a patient may be transferred from a first location to a second location, it may be desirable to transfer the patient without removing the patient's head and/or extremity from the support system. Having a mechanism for selecting and maintaining the position of the head and/or limb ensures that patients subject to the above conditions cannot change the tilting angle of the support system themselves. A further benefit to the support system is that some of the positions in which the patient's head and/or limb is positioned may be uncomfortable without support from the support system.

JP 2002 291731 A describes a subject fixing device for a medical image diagnostic apparatus allowing easy attachment and detachment to and from a top plate on which a subject is placed, capable of continuously adjusting a slant angle of the head and the jaw of the subject, capable of stably fixing the jaw and the arms in an easy attitude, and generating no metal artifact.

US 2003/159216 A1 describes a patient immobilization device that comprises a backboard having a front side and a back side, and a pair of opposing paddles slidably mounted on the backboard. The paddles are configured to move between a storage position and a support position, to support the head of a patient. Each paddle has a leg portion depending therefrom and extends through a respective slot formed in the backboard between the front and back and sides. A spanning portion of the paddle depends from the leg portion and engages the back side of the backboard to secure the paddle to the backboard. The spanning portion moves in a generally arcuate path between the storage and support positions.

US 4400820 A describes a head holder for an X-ray subject which uses a rigid U-shaped receptacle having an angulated cervical extension that fastens to a patient supporting cradle. A head restraint sheet that may be preformed to a U-shape has a tie-down strap fastened to it is wrapped about the bottom and sides of a flexible foam preformed U-shaped liner that has notches for accommodating the subject's ears. The sheet and liner are deposited in the U-shaped rigid receptacle with the head of the subject in the liner and the strap ends crossed over the forehead and attached to "Velcro" pile that is fixed on the outside of the receptacle. The resilient foam liner and flexible sheet act as a pressure feedback system which restores a head to its initial relaxed position if the head has moved. A wedge is inserted between the flexible sheet and receptacle bottom to tilt the head of the subject.

JP 2000 116645 A describes a tool for fixing the head part constituted of a non-metallic mat mounted on a head part placing part for a CT scan apparatus and an MRI apparatus, a non-metallic forehead part fixing means for supporting the forehead of the subject and a non-metallic jaw part fixing means for supporting the jaw part of the subject. In addition, a pad made of a urethane foam is respectively provided on the bottom part of the mat, a forehead contacting part of the forehead fixing means and a jaw contacting part of the jaw part fixing means.

US 2007/270683 Al describes a head restraint kit for use with a medical head device for immobilizing the head of a patient in medical research, diagnosis and operation. The head restraint kit is particularly suitable for use with a head coil for magnetic resonance imaging. The head restraint kit contains a head rest pillow for providing support to the head of a patient, a neck cushion for providing neck support, and one or more wedge cushions for providing further support for the head. The head restraint kit may also contain one or more compact square cushions which can be inserted into a space between the head and the medical head device for additional head support. The head restraint kit may further contain a cloth strap placed over the forehead of a patient for protecting and securing the head against movement.

### BRIEF DESCRIPTION

In one embodiment, a support system includes a cradle interface pad for use with an imaging system, the cradle interface pad comprising a base with a planar first side and a curved second side, opposite the first side, a first wall positioned on a first end of the base and a second wall positioned on a second end of the base, wherein the first wall, the second wall, and the planar first side of the base form a concave recess of the cradle interface pad, at least one slot extending through each of the first wall and the second wall, at least one attachment point mounted on each of the first wall and the second wall and extending towards the base, and a handle positioned on a top face of each of the first wall and the second wall, wherein the handles are configured to provide an element of the cradle interface pad for a patient to grasp with their hands during imaging to assist in moving the patient's hands, arms, and shoulders away from a region to be imaged.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present disclosure will be better understood from reading the following description of non-limiting embodiments, with reference to the attached drawings, wherein below:
FIG. 1 shows a pictorial view of an imaging system, in accordance with one or more embodiments of the present disclosure;
FIG. 2 shows a block schematic diagram of an exemplary imaging system, in accordance with one or more embodiments of the present disclosure;
FIG. 3A shows a first view of an embodiment of a cradle interface pad;
FIG. 3B shows a second view of the embodiment of the cradle interface pad;
FIG. 3C shows a third view of the embodiment of the cradle interface pad;
FIG. 4A shows a front perspective view of a first embodiment of a patient interface head pad;
FIG. 4B shows a rear perspective view of the first embodiment of a patient interface head pad;
FIG. 5A shows a front perspective view of a first embodiment of a patient interface extremity pad;
FIG. 5B shows a rear perspective view of the first embodiment of the patient interface extremity pad;
FIG. 6A shows a front perspective view of a first embodiment of an angled positioning pad;
FIG. 6B shows a cross-section view of the first embodiment of the angled positioning pad;
FIG. 7A shows a first view of a first configuration of a subject positioning kit, including the cradle interface pad, coupled to a table;
FIG. 7B shows a second view of the first configuration of the subject positioning kit coupled to the table;
FIG. 7C shows a third view of the first configuration of the subject positioning kit coupled to the table;
FIG. 8A shows a first view of a second configuration of the subject positioning kit, including the cradle interface pad;
FIG. 8B shows a second view of the second configuration of the subject positioning kit;
FIG. 9A shows a first view of a third configuration of the subject positioning kit, including the cradle interface pad;
FIG. 9B shows a second view of the third configuration of the subject positioning kit;
FIG. 10A shows a first view of a fourth configuration of the subject positioning kit, including the cradle interface pad; and
FIG. 10B shows a second view of the fourth configuration of the subject positioning kit.

### DETAILED DESCRIPTION

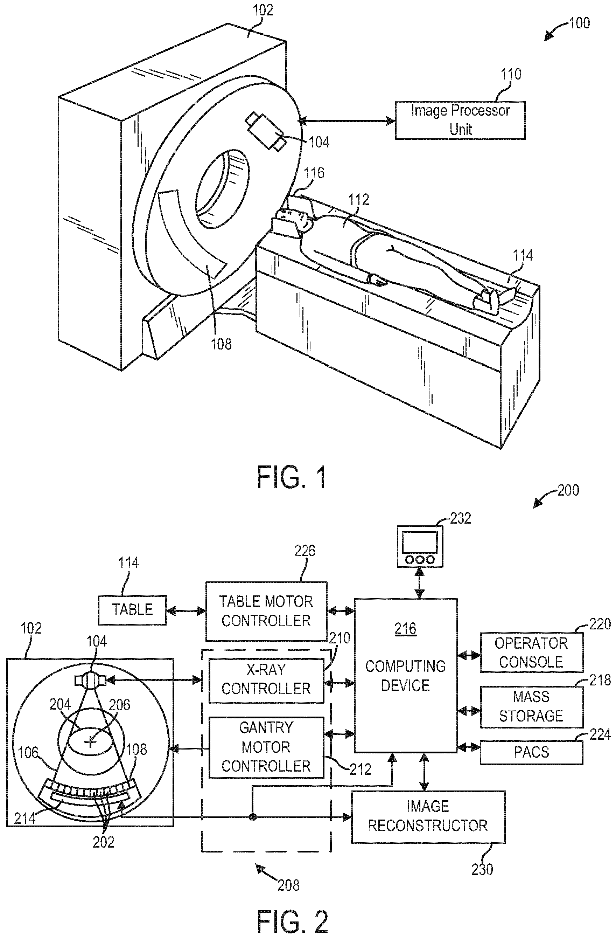

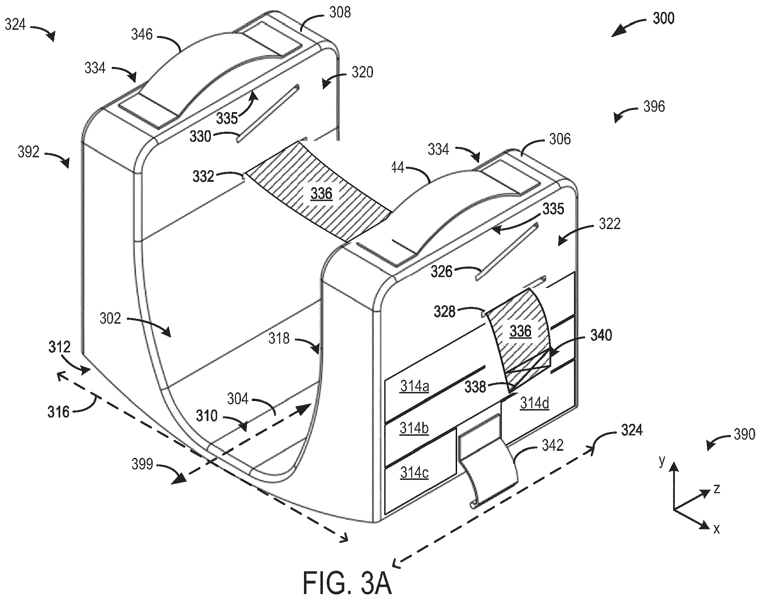

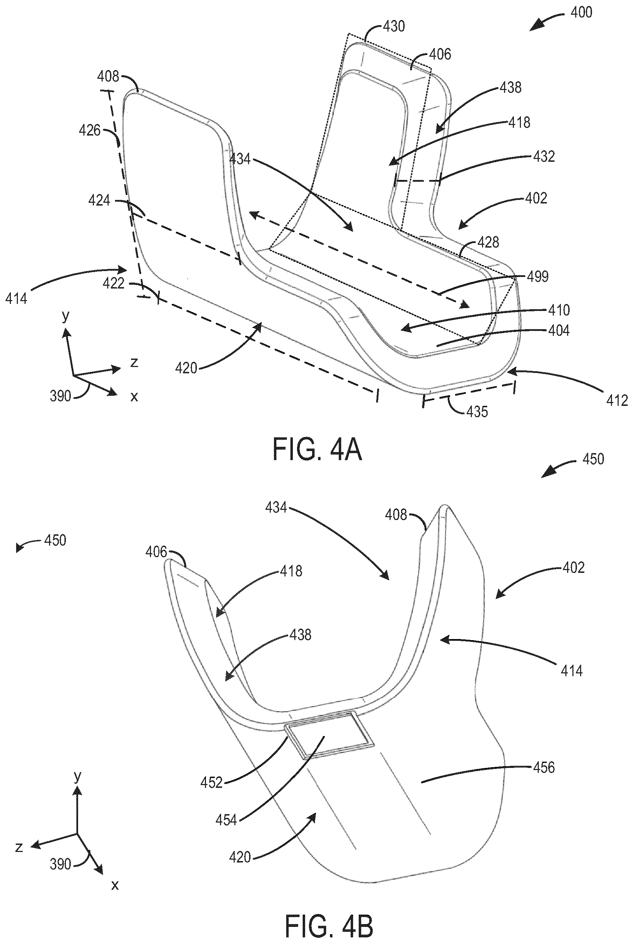

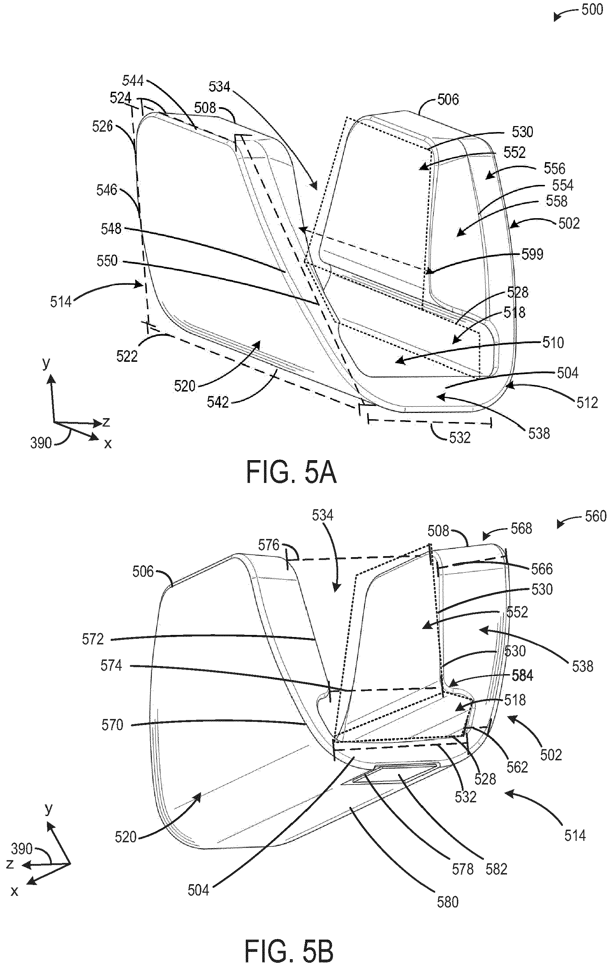

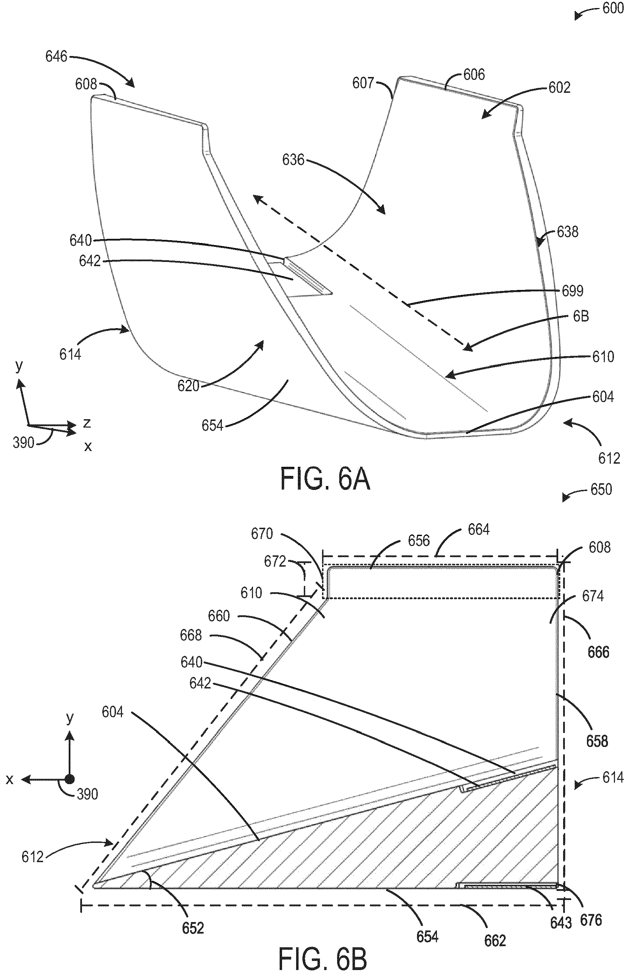

The following description relates to embodiments of a patient head and/or extremity support system. In one example, the patient head and/or extremity support system is a cradle interface pad for an imaging system, such as a CT imaging system illustrated in FIGS. 1 and 2. An embodiment of the cradle interface pad, shown in FIGS. 3A-3C, comprises a first wall and a second wall, opposite the first wall, which together form a concave recess therebetween. The first wall and the second wall each have at least one slot extending there through, at least one attachment point mounted thereon, and at least one handle positioned on a top face of each of the first wall and the second wall. The concave recess of the cradle interface pad may be configured to have a patient interface head pad, a first embodiment of which is shown in FIGS. 4A and 4B, and/or a patient interface extremity pad, a first embodiment of which is shown in FIGS. 5A and 5B, positioned therein. The embodiment of the cradle interface pad, the first embodiment of the patient interface head pad, and the first embodiment of the patient interface extremity pad may each be configured to interface with at least one angled positioning pad. A first embodiment of an angled positioning pad is shown in FIGS. 6A and 6B.

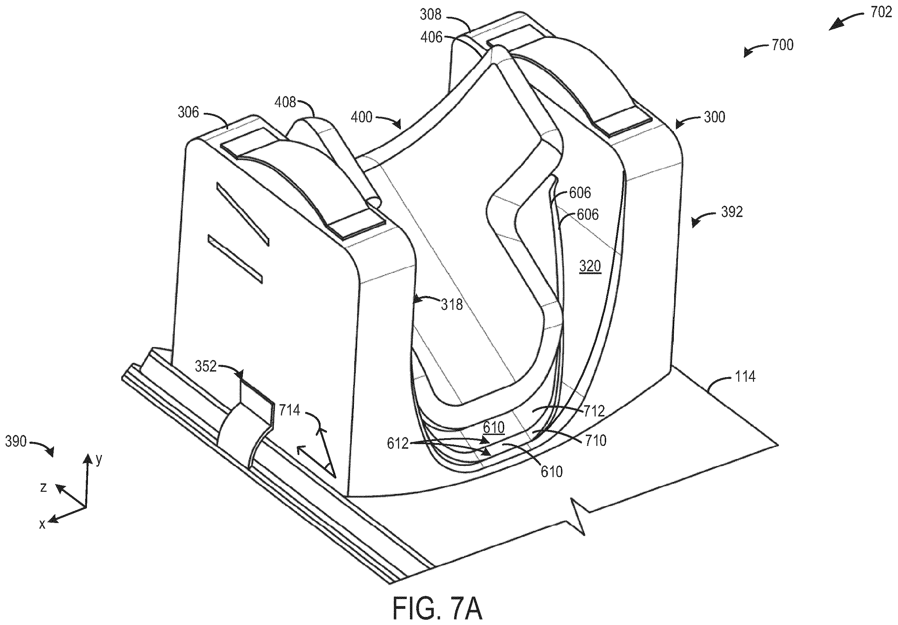

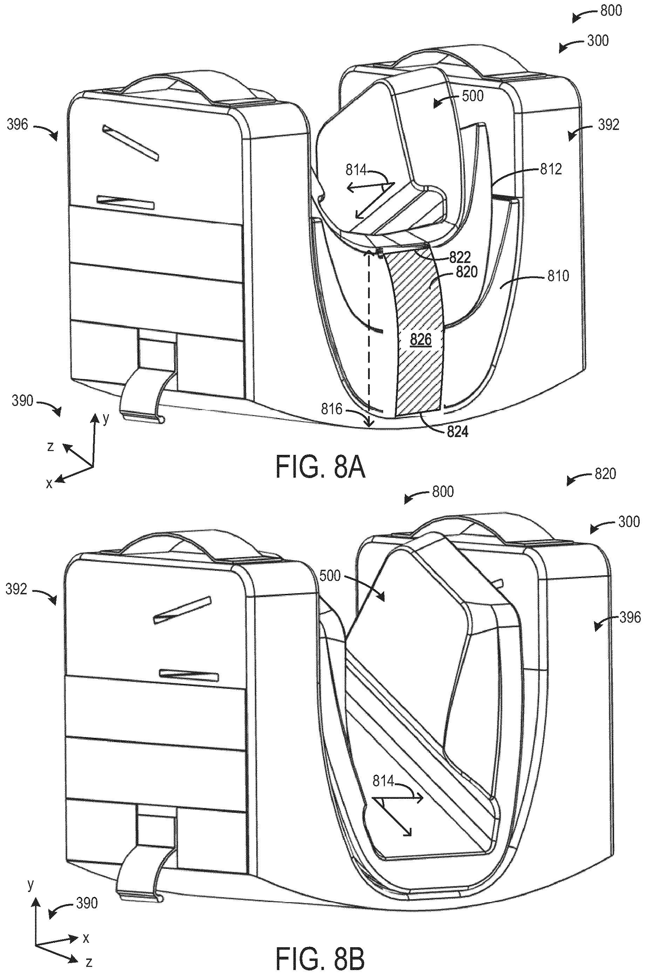

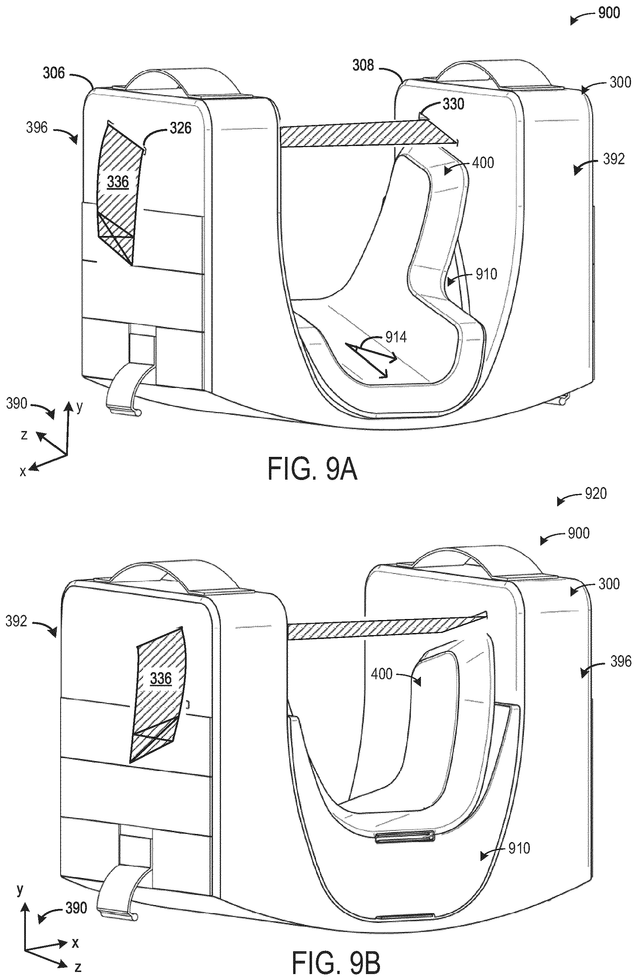

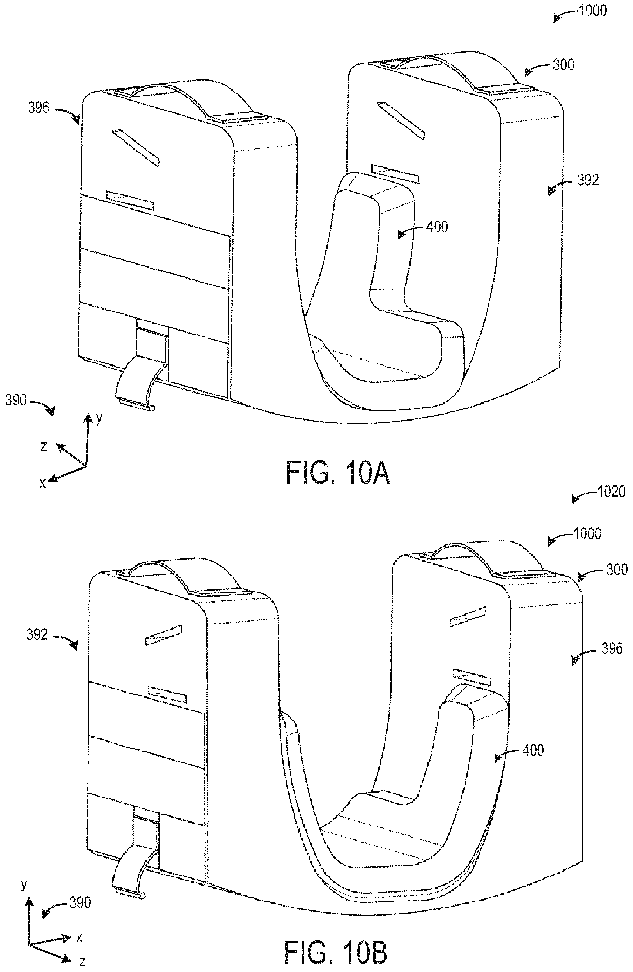

A subject positioning kit may include the cradle interface pad, at least one angled positioning pad, the patient interface head pad, and the patient interface extremity pad. The subject positioning kit may be configurable to have multiple combinations of angled positioning pads and patient interface pads positioned in the concave recess of the cradle interface pad, depending on a desired imaging anatomy (e.g., head or extremity) and angle at which to position the anatomy. FIGS. 7A-7C show a first configuration of the subject positioning kit where the cradle interface pad is coupled to a table (e.g., of a CT imaging system) via the at least one attachment points, and the cradle interface pad further has two angled positioning pads and the patient interface head pad stacked in the concave recess of the cradle interface pad to create a positive angle. A second configuration of the subject positioning kit shown in FIGS. 8A and 8B includes two angled positioning pads and the patient interface extremity pad stacked in the concave recess of the cradle interface pad to create a negative angle. A third configuration of the subject positioning kit shown in FIGS. 9A and 9B may be substantially similar to the first configuration of the cradle interface pad, where the third configuration includes one angled positioning pad and the patient interface head pad stacked in the concave recess of the cradle interface pad. A fourth configuration of the subject positioning kit shown in FIGS. 10A and 10B includes the patient interface head pad positioned in the concave recess of the cradle interface pad, creating a negligible angle. FIGS. 3A to 10B are shown to scale, however, other relative dimensions may be used if desired.

In one example, the cradle interface pad may assist a radiologist to position a patient's head and/or extremity, such as an arm, hand, wrist, leg, ankle, or foot during computed tomography (CT) x-ray examination. Previous examples include where a fixed head holder is used for an axial head scan in a CT imaging system having a tilting gantry mechanism to avoid directing x-ray radiation toward a patient's eyes. Other examples include where a head holder includes an adjustable mechanism having a lock to maintain a selected angle. The cradle interface pad of the present disclosure assists in providing desired positioning for neuro imaging in a CT imaging system and adds more benefits in cases where the gantry is fixed and unable to tilt. This may be achieved by inserting one or more of the plurality of pads into the cradle interface pad. The cradle interface pad with at least one angled positioning pad positioned in a concave recess thereof may tilt the patient's head forward or backward, depending on a position of the at least one angled positioning pad to align the brain anatomy with the scanners field of view. This minimizes radiation dose exposure to the eyes and may reduce image artifacts from dental implants. When the anatomy to be scanned is a patient head, a patient interface head pad may be positioned on top of (e.g., in face-sharing contact with) the at least one angled positioning pad which is positioned in the concave recess of the cradle interface pad. The insert (e.g., a number of angled positioning pads and a type of patient interface pad) may be exchanged on a per patient basis and/or in accordance with a desired imaging procedure, to achieve a desired vertical tilt of the imaging subject.

The cradle interface pad is sized to allow for additional pads to support additional appendages such as wrist, ankles, feet, etc., allowing those extremities to be scanned over air and reduced dose. For example, when the anatomy to be scanned is an extremity, a patient interface extremity pad may be positioned in the concave recess of the cradle interface pad and/or on top of (e.g., in face-sharing contact with) at least one angled positioning pad positioned in the concave recess of the cradle interface pad. For imaging of patient extremities, tilting the patient's extremity forward (e.g., at a positive angle, relative to the patient's body) or backward (e.g., at a negative angle, relative to the body) may enable imaging of a region of interest of the extremity which may be otherwise blocked by other anatomy or challenging to image. The cradle interface pad and other elements included in a subject positioning kit may thus be used during imaging scans to support and adjust the patient's head and/or extremity through a range of discrete angles. The cradle interface pad may further allow for additional pads to support infants or smaller animals, with the aforementioned benefits.

In some examples, the pads (e.g., angled positioning pads and patient interface pad) may be color coded for providing the user with visual recognition allowing the user to select the appropriate pad(s) quickly. In some examples, using angled pads for positioning may be preferable to existing head holder designs as they provide a range of vertical tilt, stably, and with a reduced potential of pinching. In one example, selectable angles may include 0°, 15°, 30° and 45°, however, other ranges may be utilized without departing from the scope of the present disclosure. The subject positioning kit, including the cradle interface pad, angled positioning pads, and patient interference pads, may thus be used during imaging scans to support and adjust the patient's head or extremity through a range of discrete angles.

In one example, the cradle interface pad may use a strap mechanism to capture one or more selected pads (e.g., angled positioning pads and/or patient interface pads positioned in the concave recess of the cradle interface pad) and fasten the captured pads to the cradle interface pad, such that once the support assembly is properly positioned, the configuration is secured and the patient is not able to change the angle. Additionally, the cradle interface pad may include a pair of slots on the opposing walls. The opposing slots provide an opening through which an additional strap may be inserted for securing a position of the patient's head or extremity. This may reduce motion of the patient's head and/or extremity to a position other than that of the desired position used for clear imaging, such as for patients subject to involuntary movements, who may be combative, and/or for positions which may be uncomfortable to maintain without support from a support system, such as the cradle interface pad described herein. Additionally, handles positioned on a top face of each of the first wall and the second wall of the cradle interface pad may provide an element of the cradle interface pad for the patient to grasp with their hands during imaging, which may assist in moving the patient's hands, arms, and shoulders away from a region to be imaged while also providing a comfortable position for the patient during imaging. The cradle interface pad and other elements included in the subject positioning kit, such as a positioning strap and side handles, may enable secured positioning of a patient's head and/or extremity, meaning that once a patient's head and/or extremity is properly positioned, the angle is locked in place and the patient is not able to change the angle by lifting their head and/or extremity out of the cradle interface pad.

The cradle interface pad is free of metallic or sharp edge components within an imaging range. This is accomplished via at least one attachment point arranged on the first wall and/or the second wall of the cradle interface pad and extending towards a curved base of the cradle interface pad, out of an imaging range of a patient's head or neck that may be imaged by the CT imaging system. The at least one attachment point is configured to interface with a patient table, for example, of a CT imaging system on which a patient rests during imaging. The at least one attachment point may be configured as a hook which slides into a track of the patient table, eliminating a locking mechanism from the cradle interface pad which, when included in other support systems, may be a pinch point or other source of metal which may interfere with imaging.

Having a mechanism for selecting and maintaining a head and/or extremity position ensures that patients subject to the above conditions cannot change the tilting angle of the support system themselves. A further benefit to the support system for a patient head and/or extremity is that some of the positions in which the patient's head and/or extremity is positioned via the support system may be uncomfortable without support from the support system. Additionally, in emergency situations where a patient may be transferred from a first location to a second location, it may be desirable to transfer the patient without removing the patient's head and/or extremity from the support system. The at least one attachment points of the cradle interface pad may enable the cradle interface pad to be coupled to and uncoupled from a table having a track configured to receive the cradle interface pad, which may enable the cradle interface pad to be moved between tables or other surfaces on which a patient may rest without removing the patient head and/or extremity from the cradle interface pad.

FIGS. 3A through 10B show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example.

FIG. 1 illustrates an exemplary CT imaging system 100 configured for CT imaging. Particularly, the CT imaging system 100 is configured to image a subject 112 such as a patient, an inanimate object, one or more manufactured parts, and/or foreign objects such as dental implants, stents, and/or contrast agents present within the body. In one embodiment, the CT imaging system 100 includes a gantry 102, which in turn, may further include at least one x-ray source 104 configured to project a beam of x-ray radiation 106 (see FIG. 2) for use in imaging the subject 112 laying on a table 114. Specifically, the x-ray source 104 is configured to project the x-ray radiation beams 106 towards a detector array 108 positioned on the opposite side of the gantry 102. Although FIG. 1 depicts only a single x-ray source 104, in certain embodiments, multiple x-ray sources and detectors may be employed to project a plurality of x-ray radiation beams 106 for acquiring projection data at different energy levels corresponding to the patient. In some embodiments, the x-ray source 104 may enable dual-energy gemstone spectral imaging (GSI) by rapid peak kilovoltage (kVp) switching. In some embodiments, the x-ray detector employed is a photon-counting detector which is capable of differentiating x-ray photons of different energies. In other embodiments, two sets of x-ray sources and detectors are used to generate dual-energy projections, with one set at low-kVp and the other at high-kVp. It should thus be appreciated that the methods described herein may be implemented with single energy acquisition techniques as well as dual energy acquisition techniques.

In certain embodiments, the CT imaging system 100 further includes an image processor unit 110 configured to reconstruct images of a target volume of the subject 112 using an iterative or analytic image reconstruction method. For example, the image processor unit 110 may use an analytic image reconstruction approach such as filtered back projection (FBP) to reconstruct images of a target volume of the patient. As another example, the image processor unit 110 may use an iterative image reconstruction approach such as advanced statistical iterative reconstruction (ASIR), conjugate gradient (CG), maximum likelihood expectation maximization (MLEM), model-based iterative reconstruction (MBIR), and so on to reconstruct images of a target volume of the subject 112. As described further herein, in some examples the image processor unit 110 may use both an analytic image reconstruction approach such as FBP in addition to an iterative image reconstruction approach.

In some CT imaging system configurations, an x-ray source projects a cone-shaped x-ray radiation beam which is collimated to lie within an X-Y-Z plane of a Cartesian coordinate system and generally referred to as an "imaging plane." The x-ray radiation beam passes through an object being imaged, such as the patient or subject. The x-ray radiation beam, after being attenuated by the object, impinges upon an array of detector elements. The intensity of the attenuated x-ray radiation beam received at the detector array is dependent upon the attenuation of a radiation beam by the object. Each detector element of the array produces a separate electrical signal that is a measurement of the x-ray beam attenuation at the detector location. The attenuation measurements from all the detector elements are acquired separately to produce a transmission profile.

In some CT imaging systems, the x-ray source and the detector array are rotated with a gantry within the imaging plane and around the object to be imaged such that an angle at which the radiation beam intersects the object constantly changes. A group of x-ray radiation attenuation measurements, e.g., projection data, from the detector array at one gantry angle is referred to as a "view." A "scan" of the object includes a set of views made at different gantry angles, or view angles, during one revolution of the x-ray source and detector. It is contemplated that the benefits of the methods described herein accrue to medical imaging modalities other than CT, so as used herein the term "view" is not limited to the use as described above with respect to projection data from one gantry angle. The term "view" is used to mean one data acquisition whenever there are multiple data acquisitions from different angles, whether from a CT, positron emission tomography (PET), or single-photon emission CT (SPECT) acquisition, and/or any other modality including modalities yet to be developed as well as combinations thereof in fused embodiments.

The projection data is processed to reconstruct an image that corresponds to a two-dimensional slice taken through the object or, in some examples where the projection data includes multiple views or scans, a three-dimensional rendering of the object. One method for reconstructing an image from a set of projection data is referred to in the art as the filtered back projection technique. Transmission and emission tomography reconstruction techniques also include statistical iterative methods such as maximum likelihood expectation maximization (MLEM) and ordered-subsets expectation-reconstruction techniques as well as iterative reconstruction techniques. This process converts the attenuation measurements from a scan into integers called "CT numbers" or "Hounsfield units," which are used to control the brightness of a corresponding pixel on a display device.

To reduce the total scan time, a "helical" scan may be performed. To perform a "helical" scan, the patient is moved while the data for the prescribed number of slices is acquired. Such a system generates a single helix from a cone beam helical scan. The helix mapped out by the cone beam yields projection data from which images in each prescribed slice may be reconstructed.

As used herein, the phrase "reconstructing an image" is not intended to exclude embodiments of the present disclosure in which data representing an image is generated but a viewable image is not. Therefore, as used herein, the term "image" broadly refers to both viewable images and data representing a viewable image. However, many embodiments generate (or are configured to generate) at least one viewable image.

In one example, the table 114 may include a cradle interface pad 116 which may be combined with one or more of a plurality of pads to position and/or tilt an anatomy for imaging. In the example of FIG. 1, the cradle interface pad 116 is holding a head. In other examples, the cradle interface pad 116 may support an extremity, such as an arm, wrist, leg, and/or ankle. Previous examples of head holders may be bulky, complex, or subject to potential pinching of the patient and/or a medical provider working with the cradle interface pad 116. Furthermore, the previous examples demand a greater amount of operator effort and the process may become cumbersome and time consuming. If a patient moves their head when positioned in the head holder of a previous example, the adjustment mechanism may become free and a position of the head holder may change. This may result in the patient sliding off the table and into a CT gantry. Additionally, in previous examples, a different support system may be used to position a patient head than is used to position a patient extremity. Thus, a head holder may be removed from the table 114 and an extremity holder may be added to the table 114 when switching from head imaging to extremity imaging, which may be cumbersome and time consuming. Previous examples of head holders which offer different configurable angles may be bulky, complex, or lacking auto-regulating features. Furthermore, the previous examples demand a greater amount of operator effort and the process may become cumbersome and time consuming. Additionally, in the previous examples, an operator may use both hands to adjust the head holder. If a patient moves their head when positioned in the head holder of a previous example, the adjustment mechanism may become free and an angle at which the patient's head and/or extremity is positioned may change. This may result in the patient being exposed to an undesired level of x-ray radiation, such as exposing a patient's eyes and/or organs outside of a region of interest to x-ray radiation. Further, adjustment of the head holder to provide different angles at which a patient's head and/or extremity may be positioned may be complex and include use of mechanical lock and pin mechanisms, for example.

In one example of the present disclosure, as will be described below in greater detail with respect to FIG. 3A and the subsequent figures, the cradle interface pad 116 may be configured to support a patient's head and/or extremity at a range of angles. The cradle interface pad may be used in combination with a patient interface pad, such as a patient interface head pad or a patient interface extremity pad, and optionally at least one angled positioning pad positioned in a concave recess of the cradle interface pad. Using of various combinations of pads may provide a range of tilts or angles for an imaging procedure. Further, the cradle interface pad may include at least one attachment point which enables coupling of the cradle interface pad to a table, such as the table 114 of the CT imaging system 100. Configuration of the at least one attachment point as a hook enables the cradle interface pad to easily be coupled to the table and to be positioned at points along a length of the table, which may allow for patient positioning which may be challenging, uncomfortable, and/or impractical when attempted using prior support systems. The cradle interface pad includes a curved base, allowing the cradle interface pad to be positioned in face-sharing contact with the table, which may reduce undesired angling of a patient head and/or extremity. A strap may further be used in combination with the cradle interface pad in some examples to secure a patient anatomy in the cradle interface pad.

FIG. 2 illustrates an exemplary imaging system 200 similar to the CT imaging system 100 of FIG. 1. In accordance with aspects of the present disclosure, the imaging system 200 is configured for imaging a subject 204 (e.g., the subject 112 of FIG. 1). In one embodiment, the imaging system 200 includes the detector array 108 (see FIG. 1). The detector array 108 further includes a plurality of detector elements 202 that together sense the x-ray radiation beam 106 (see FIG. 2) that pass through the subject 204 (such as a patient) to acquire corresponding projection data. Accordingly, in one embodiment, the detector array 108 is fabricated in a multi-slice configuration including the plurality of rows of cells or detector elements 202. In such a configuration, one or more additional rows of the detector elements 202 are arranged in a parallel configuration for acquiring the projection data.

In certain embodiments, the imaging system 200 is configured to traverse different angular positions around the subject 204 for acquiring desired projection data. Accordingly, the gantry 102 and the components mounted thereon may be configured to rotate about a center of rotation 206 for acquiring the projection data, for example, at different energy levels. Alternatively, in embodiments where a projection angle relative to the subject 204 varies as a function of time, the mounted components may be configured to move along a general curve rather than along a segment of a circle.

As the x-ray source 104 and the detector array 108 rotate, the detector array 108 collects data of the attenuated x-ray beams. The data collected by the detector array 108 undergoes pre-processing and calibration to condition the data to represent the line integrals of the attenuation coefficients of the scanned subject 204. The processed data are commonly called projections.

In some examples, the individual detectors or detector elements 202 of the detector array 108 may include photon-counting detectors which register the interactions of individual photons into one or more energy bins. It should be appreciated that the methods described herein may also be implemented with energy-integrating detectors.

The acquired sets of projection data may be used for basis material decomposition (BMD). During BMD, the measured projections are converted to a set of material-density projections. The material-density projections may be reconstructed to form a pair or a set of material-density map or image of each respective basis material, such as bone, soft tissue, and/or contrast agent maps. The density maps or images may be, in turn, associated to form a volume rendering of the basis material, for example, bone, soft tissue, and/or contrast agent, in the imaged volume.

Once reconstructed, the basis material image produced by the imaging system 200 reveals internal features of the subject 204, expressed in the densities of two basis materials. The density image may be displayed to show these features. In traditional approaches to diagnosis of medical conditions, such as disease states, and more generally of medical events, a radiologist or physician would consider a hard copy or display of the density image to discern characteristic features of interest. Such features might include lesions, sizes and shapes of particular anatomies or organs, and other features that would be discernable in the image based upon the skill and knowledge of the individual practitioner.

In one embodiment, the imaging system 200 includes a control mechanism 208 to control movement of the components such as rotation of the gantry 102 and the operation of the x-ray source 104. In certain embodiments, the control mechanism 208 further includes an x-ray controller 210 configured to provide power and timing signals to the x-ray source 104. Additionally, the control mechanism 208 includes a gantry motor controller 212 configured to control a rotational speed and/or position of the gantry 102 based on imaging requirements.

In certain embodiments, the control mechanism 208 further includes a data acquisition system (DAS) 214 configured to sample analog data received from the detector elements 202 and convert the analog data to digital signals for subsequent processing. The DAS 214 may be further configured to selectively aggregate analog data from a subset of the detector elements 202 into so-called macro-detectors, as described further herein. The data sampled and digitized by the DAS 214 is transmitted to a computer or computing device 216. In one example, the computing device 216 stores the data in a storage device 218. The storage device 218, for example, may include a hard disk drive, a floppy disk drive, a compact disk-read/write (CD-R/W) drive, a Digital Versatile Disc (DVD) drive, a flash drive, and/or a solid-state storage drive.

Additionally, the computing device 216 provides commands and parameters to one or more of the DAS 214, the x-ray controller 210, and the gantry motor controller 212 for controlling system operations such as data acquisition and/or processing. In certain embodiments, the computing device 216 controls system operations based on operator input. The computing device 216 receives the operator input, for example, including commands and/or scanning parameters via an operator console 220 operatively coupled to the computing device 216. The operator console 220 may include a keyboard (not shown) or a touchscreen to allow the operator to specify the commands and/or scanning parameters.

Although FIG. 2 illustrates only one operator console 220, more than one operator console may be coupled to the imaging system 200, for example, for inputting or outputting system parameters, requesting examinations, plotting data, and/or viewing images. Further, in certain embodiments, the imaging system 200 may be coupled to multiple displays, printers, workstations, and/or similar devices located either locally or remotely, for example, within an institution or hospital, or in an entirely different location via one or more configurable wired and/or wireless networks such as the Internet and/or virtual private networks, wireless telephone networks, wireless local area networks, wired local area networks, wireless wide area networks, wired wide area networks, etc.

In one embodiment, for example, the imaging system 200 either includes, or is coupled to, a picture archiving and communications system (PACS) 224. In an exemplary implementation, the PACS 224 is further coupled to a remote system such as a radiology department information system, hospital information system, and/or to an internal or external network (not shown) to allow operators at different locations to supply commands and parameters and/or gain access to the image data.

The computing device 216 uses the operator-supplied and/or system-defined commands and parameters to operate a table motor controller 226, which in turn, may control the table 114 which may be a motorized table. Specifically, the table motor controller 226 may move the table 114 for appropriately positioning the subject 204 in the gantry 102 for acquiring projection data corresponding to the target volume of the subject 204.

As previously noted, the DAS 214 samples and digitizes the projection data acquired by the detector elements 202. Subsequently, an image reconstructor 230 uses the sampled and digitized x-ray data to perform high-speed reconstruction. Although FIG. 2 illustrates the image reconstructor 230 as a separate entity, in certain embodiments, the image reconstructor 230 may form part of the computing device 216. Alternatively, the image reconstructor 230 may be absent from the imaging system 200 and instead the computing device 216 may perform one or more functions of the image reconstructor 230. Moreover, the image reconstructor 230 may be located locally or remotely, and may be operatively connected to the imaging system 200 using a wired or wireless network. Particularly, one exemplary embodiment may use computing resources in a "cloud" network cluster for the image reconstructor 230.

In one embodiment, the image reconstructor 230 stores the images reconstructed in the storage device 218. Alternatively, the image reconstructor 230 may transmit the reconstructed images to the computing device 216 for generating useful patient information for diagnosis and evaluation. In certain embodiments, the computing device 216 may transmit the reconstructed images and/or the patient information to a display or display device 232 communicatively coupled to the computing device 216 and/or the image reconstructor 230. In some embodiments, the reconstructed images may be transmitted from the computing device 216 or the image reconstructor 230 to the storage device 218 for short-term or long-term storage.

Though a CT system is described by way of example, it should be understood that the present technology may also be used on other imaging modalities, such as x-ray imaging systems, magnetic resonance imaging (MRI) systems, nuclear medicine imaging systems, positron emission tomography (PET) imaging systems, single-photon emission computed tomography (SPECT) imaging systems, ultrasound imaging systems, and combinations thereof (e.g., multi-modality imaging systems, such as PET/CT or PET/MR imaging systems). The present discussion of a CT imaging modality is provided merely as an example of one suitable imaging modality.

The cradle interface pad may be used with an imaging system having a table, such as illustrated with respect to FIGS. 1-2. In some examples, the cradle interface pad may be used in combination with one or more of a plurality of interchangeable pads, including patient interface pads, such as a patient interface head pad and/or a patient interface extremity pad, and optionally at least one angled positioning pad, to adjust a tilt angle of a head or extremity of a subject relative to a horizontal axis (e.g., see coordinate system 390 below).

Turning to FIGS. 3A-3C, various views of an example of a cradle interface pad 300 are shown. As described above, a patient such as subject 112 of FIG. 1 may have their head positioned on the cradle interface pad 300 to maintain a desired tilt angle of their head for imaging. CT imaging system 100 represents one exemplary usage of the cradle interface pad 300. As such, the cradle interface pad 300 may be a non-limiting example of the cradle interface pad 116 of FIG. 1.

A coordinate system 390 is shown comprising three-axes, namely an x-axis parallel to a horizontal direction, a y-axis parallel to a vertical direction, and a z-axis perpendicular to each of the x- and y-axes. For reference, the coordinate system 390 is included in FIGS. 3A-6B and FIGS. 8-10B. The cradle interface pad 300 comprises a central axis 399, which lies in an x-z plane. The cradle interface pad 300 includes a first end 392 and a second end 396. As further described with respect to FIGS. 7A-10B, when positioned in a CT system, such as the CT imaging system 100 of FIG. 1, the first end 392 may face towards or away from the gantry 102.

The cradle interface pad 300 is formed of a base 304 with a first wall 306 and a second wall 308 extending therefrom. The base 304 may be a curved base which is substantially planar on a first side 310 and curved on a second side 312, where an angle of curvature of the second side 312 is complementary to an angle of curvature of a table on which the cradle interface pad 300 may be placed, such as the table 114 of FIG. 1. Each of the first wall 306 and the second wall 308 may be parallel to a y-axis, with respect to the coordinate system 390. The first wall 306 and the second wall 308 may extend from opposite edges of the base 304 such that the first wall 306 and the second wall 308 may function as boundaries which block a patient from moving their head and/or extremity laterally (e.g., along an x-axis, with respect to the coordinate system 390) out of the cradle interface pad 300. For example, the first wall 306 and the second wall 308 are positioned on opposite ends of the base 304 along a length 316 of the cradle interface pad 300, where the first wall 306 is positioned on a first end and the second wall 308 is positioned on a second end, opposite the first end. The second side 312 of the base 304 curves along the length 316, such that the second side 312 of the base 304 may rest on a flat surface (e.g., a non-curved table) along the central axis 399 and the first wall 306 and the second wall 308 may not rest on the flat surface. In some examples, the first wall 306, the second wall 308, and the base 304 of the cradle interface pad 300 are formed of inflexible (e.g., rigid) material, such as carbon fiber or plastic. In other examples, the first wall 306, the second wall 308, and the base 304 of the cradle interface pad 300 may be formed of a foam material including rubber, polyethylene, polyurethane, or polystyrene based foams. The cradle interface pad 300 may further be formed of or coated with a non-porous material, which enables cleaning and disinfection of the cradle interface pad 300, such as between patient scans. Cradle interface pad 300 elements may be formed of other materials without departing from the scope of the present disclosure.

A concave recess 302 is formed between the first wall 306 and the second wall 308, such that the concave recess 302 may receive a subject interface pad and, optionally, at least one angled positioning pad, as further described with respect to FIGS. 4A-10B. Each of a first interior side 318 of the first wall 306 and a second interior side 320 of the second wall 308 may be curved to form the concave recess 302. Described another way, the first wall 306 and the second wall 308 are each curved extensions from the first side 310 of the base 304 and flatten as they extend upward. A configuration of the first wall 306 may mirror a configuration of the second wall 308. Thus, the concave recess 302 may be substantially planar along the central axis 399 and curve laterally to form a U-shape.

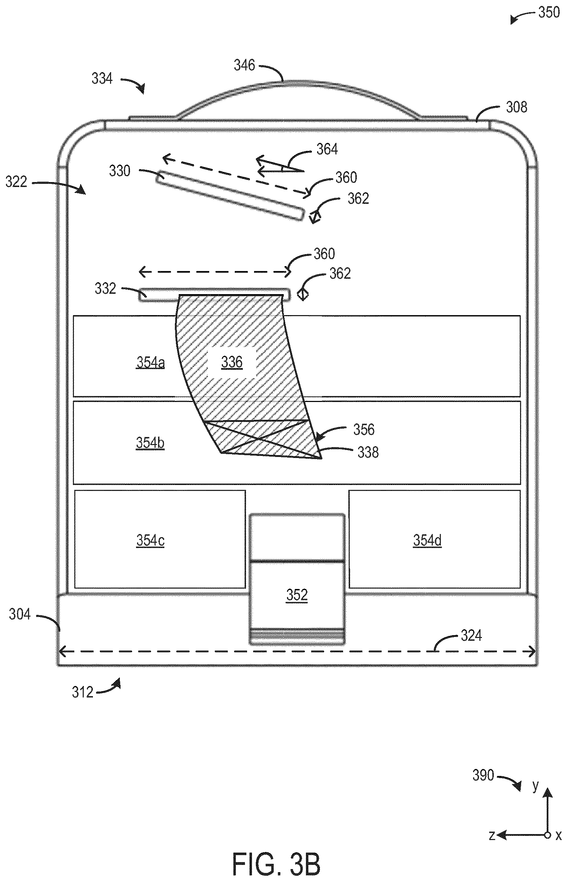

The cradle interface pad 300 further includes an outer surface 322, which may be an outward facing surface, e.g., an exterior, of the first wall 306, the base 304 (e.g., the second side 312 of the base 304), and the second wall 308. In some examples, the outer surface 322 may include a fastening portion with a fastening material adhered thereto. For example, the fastening material may extend along a width 324 of each of the first wall 306 and the second wall 308. The width 324 may be less than the length 316 of the cradle interface pad 300. In some examples, the coupling material may include multiple panels of coupling material. As shown in the example of FIG. 3A, the first wall 306 includes a first panel 314a, a second panel 314b, a third panel 314c, and a fourth panel 314d of fastening material. The second wall 308 is similarly configured with panels of fastening material, as described with respect to FIG. 3B. The fastening material may enable coupling of the positioning strap to the cradle interface pad 300, as further described herein and with respect to FIGS. 9A-10B.

Each of the first wall 306 and the second wall 308 include at least one slot extending there through, e.g., from the outer surface 322 to the first interior side 318 of the concave recess 302 and from the outer surface 322 to the second interior side 320 of the concave recess 302. In the example shown in FIGS. 3A-10B, the cradle interface pad 300 includes a pair of slots on each of the first wall 306 and the second wall 308. The first wall 306 includes a first slot 326 positioned above a second slot 328. The second wall 308 also includes a first slot, here a third slot 330, positioned above a second slot, here a fourth slot 332. The third slot 330 and the fourth slot 332 are oriented directly opposite the central axis 399 from the first slot 326 and the second slot 328, respectively. The second slot 328 and the fourth slot 332 are each parallel to a top edge 335 of the first wall 306 and the second wall 308, respectively. The first slot 326 and the third slot 330 are each positioned at a non-zero angle, relative to the top edge 335 of the first wall 306 and the second wall 308, respectively, as further described with respect to FIG. 3B. As further described with respect to FIG. 3B, each of the first slot 326, the second slot 328, the third slot 330, and the fourth slot 332 have a first length and a first height. In some examples of the cradle interface pad 300, the first wall 306 and the second wall 308 may have more than or less than two slots, so long as each of the first wall 306 and the second wall 308 have an equal number of slots.

Configurations of the slots, including the first length, the first width, and an angulation (e.g., parallel with the top edge 335 of the respective wall or a non-zero angle relative to the top edge 335 of the respective wall) are such that a positioning strap 336 may extend through a pair of slots (e.g., the first slot 326 and the third slot 330 or the second slot 328 and the fourth slot 332). In some examples, the positioning strap 336 may be inserted through the first slot 326 and the third slot 330 for securing a patient's head or extremity when positioned at a first angle. In other examples, such as the example shown in FIGS. 3A-3C, the positioning strap 336 may be inserted through the second slot 328 and the fourth slot 332 for securing a patient's head or extremity when positioned at a second angle. The positioning strap 336 may include a receiving portion 338 that fastens the positioning strap 336 to the fastening material of the fastening portion of the outer surface 322 of the cradle interface pad 300. The positioning strap 336 may include a receiving portion 338 at both a first strap end 340 and a second strap end, opposite the first strap end, as shown in FIG. 3B. For example, the receiving portion 338 of the positioning strap 336 may be a contrasting material with respect to the fastening material of the fastening portion of the cradle interface pad 300 and may be coupled to the second panel 314b of fastening material on at least one of the first wall 306 and the second wall 308. For example, the receiving portion 338 of the positioning strap 336 may be coupled to at least one of the first panel 314a, the second panel 314b, the third panel 314c, and the fourth panel 314d on the first wall 306 and may be coupled to at least one of the first panel 314a, the second panel 314b, the third panel 314c, and the fourth panel 314d on the second wall 308. In some examples, the receiving material of the receiving portion 338 and the fastening material of the first panel 314a, the second panel 314b, the third panel 314c, and the fourth panel 314d may be a hook and loop fastener. The positioning strap 336 may be formed of nylon or other bendable/foldable material, and the receiving material of the receiving portions may be mounted on the material of the positioning strap 336.

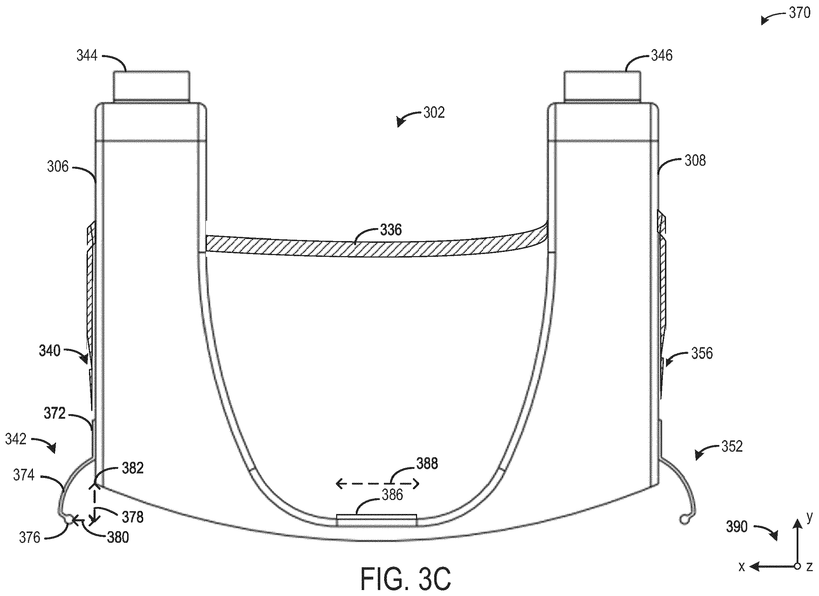

At least one attachment point is mounted on each of the first wall 306 and the second wall 308 and extends towards the second side 312 of the base 304. In the example shown in FIG. 3A, a first attachment point 342 is mounted on the first wall 306. It is to be understood that a second attachment point is mounted on the second wall 308 in a position similar to that of the first attachment point 342, as shown in FIGS. 3B and 3C. As further described with respect to FIGS. 3C and 7A-7C, a hook configuration of each of the at least one attachment points enables the at least one attachment point to interface with a track of the table on which the cradle interface pad 300 is positioned, such as the table 114 of FIG. 1.

The cradle interface pad 300 further includes a first handle 344 positioned on a top face 334 of the first wall 306 and a second handle 346 positioned on the top face 334 of the second wall 308. Each of the first handle 344 and the second handle 346 may be equivalently configured and positioned in such a way that a patient may grasp onto one or both of the handles when the patient's head is positioned in the concave recess 302 of the cradle interface pad 300. By grasping onto at least one of the first handle 344 and the second handle 346, the patient's arms may be positioned over their head in such a way that imaging of a region of interest may be unhindered by the patient's arms.

FIG. 3B shows a side profile 350 of the cradle interface pad 300, looking down the x-axis, with respect to the coordinate system 390. The side profile 350 shows the second wall 308 with the third slot 330, the fourth slot 332, and the second handle 346 as described with respect to FIG. 3A. As briefly described with respect to FIG. 3A, the second wall 308 is configured with fastening material like the fastening material of the first wall 306. The second wall 308 includes a fifth panel 354a, a sixth panel 354b, a seventh panel 354c, and an eighth panel 354d of fastening material. As shown in FIG. 3B, the positioning strap 336 includes a receiving portion 338 at a second strap end 356. The receiving portion 338 on the second strap end 356 may be used to couple the positioning strap 336 to at least one of the fifth panel 354a, the sixth panel 354b, the seventh panel 354c, and the eighth panel 354d of fastening material. In the example shown in FIG. 3B, the positioning strap 336 is coupled to the sixth panel 354b.

As described with respect to FIG. 3A, each of the first wall 306 and the second wall 308 are configured with at least one slot through which the positioning strap 336 may be passed to help position a patient head and/or extremity in the concave recess 302 of the cradle interface pad 300. In the example shown in FIGS. 3A-3C, each of the first slot 326, the second slot 328, the third slot 330, and the fourth slot 332 have a slot length 360 and a slot height 362, where the slot length 360 is greater than the slot height 362. In other examples, a slot height may be less or equal to a slot length, and the slot height and the slot length may be different for each slot and/or different for each set of slots (e.g., a first set of slots on the first wall 306 and a second set of slots on the second wall 308), so long as the slots are sized such that the positioning strap 336 may be passed there through. As described with respect to FIG. 3A, the first slot 326 and the third slot 330 are each positioned at a non-zero angle, relative to the top of the first wall 306 and the second wall 308, respectively. For example, the first slot 326 and the third slot 330 are positioned at an angle 364.

Additionally, the second wall 308 has a second attachment point 352 coupled thereto, where the second attachment point 352 may have the same configuration as the first attachment point 342 coupled to the first wall 306. Configuration of the first attachment point 342 and the second attachment point 352 are further described with respect to FIG. 3C and FIGS. 7A-7C.

FIG. 3C shows a back profile 370 of the cradle interface pad 300, looking down the z-axis, with respect to the coordinate system 390. The back profile 370 shows the first wall 306, the second wall 308, and the base 304. The positioning strap 336 as shown is threaded through the second slot 328 and the fourth slot 332 and coupled to the cradle interface pad 300 at the second panel 314b of fastening material of the first wall 306 and the sixth panel 354b of the second wall 308. In other examples, the positioning strap 336 may be threaded through the first slot 326 and the third slot 330 and/or be coupled to the cradle interface pad 300 via at least one of the first panel 314a, the third panel 314c, and the fourth panel 314d of the first wall 306, and at least one of the fifth panel 354a, the seventh panel 354c, and the eighth panel 354d of the second wall 308.

The back profile 370 further shows the first attachment point 342 on the first wall 306 and the second attachment point 352 on the second wall 308. In some examples of the cradle interface pad 300, each of the first wall 306 and the second wall 308 may have more than one attachment point positioned thereon. As briefly described with respect to FIGS. 3A and 3B, the first attachment point 342 and the second attachment point 352 have the same configuration in some examples. For example, each of the first attachment point 342 and the second attachment point 352 is configured to interface with a track of a table, such as the table 114 of the CT imaging system 100 of FIG. 1. A configuration of the first attachment point 342 and the second attachment point 352 are described herein with respect to the first attachment point 342, for brevity, though it is to be understood that the second attachment point 352 has the same configuration and be similarly mounted on the second wall 308, in some examples.

The configuration of the first attachment point 342 may include a mounting section 372, a curved extension 374 coupled to the mounting section 372, and a hook end 376 coupled to the curved extension 374. In some examples, the first attachment point 342 is formed as a single, continuous piece. The mounting section 372 may couple the first attachment point 342 to the outer surface 322 of the first wall 306. As shown in FIG. 3A, the first attachment point 342 may be positioned between the third panel 314c and the fourth panel 314d, below the second panel 314b. As shown in FIGS. 3A and 3C, the curved extension 374 of the first attachment point extends away from the first wall 306 (e.g., along the x-axis, with respect to the coordinate system 390 and towards the base 304 of the cradle interface pad 300. The hook end 376 is angled towards the first wall 306 and has an end diameter which is greater than a width of the curved extension 374 and the mounting section 372. As further described with respect to FIGS. 7A-7C, the hook end 376 may be positioned in a track of a table, such as the table 114 of the CT imaging system 100 of FIG. 1, to couple the cradle interface pad 300 to the table. A first distance 378 between a bottom edge 382 of the first wall 306 and the hook end 376, as well as a second distance 380 between the bottom edge 382 of the first wall 306 and the hook end 376 may be sized to accommodate a table edge, such as an edge of the table 114 of the CT imaging system 100 of FIG. 1, therebetween.

The cradle interface pad 300 may be an element of a subject positioning kit used to position and secure a patient head and/or extremity at a desired angle for imaging, such as performed by a CT imaging device. The subject positioning kit may further include at least one angled positioning pad, a patient interface head pad, and a patient interface extremity pad. In some embodiments, the subject positioning kit includes a securing strap for coupling at least one of an angled positioning pad, the patient interface head pad, and the patient interface extremity pad to the cradle interface pad 300.

In some examples, the cradle interface pad 300 may have a receiving material 386 in the concave recess 302. The receiving material 386 may be made of a hook and loop fastener or a similar fastening material which is complementary to a fastening material, as further described herein. In some examples, the receiving material 386 may extend a planar width 388 and the width 324 (as described with respect to FIG. 3B) of the first side 310 of the base 304. In other examples, the cradle interface pad 300 may include a first receiving material portion adjacent to the first end 392 and a second receiving material portion adjacent to the second end 396, where the first receiving material portion and the second receiving material portion are not continuous along the length of the base 304 of the cradle interface pad 300. In this way, stacking pads such as an angled positioning pad and/or a patient interface pad may be coupled to the cradle interface pad 300 via a fastening material of the stacking pad which is complementary to the receiving material 386.

FIG. 4A through FIG. 6B illustrate first, second, and third examples, respectively, of stacking pads which may be included in the subject positioning kit. In one example, the stacking pads may be used singly or in combination, e.g., stacked, to support a head or extremity of a patient. FIG. 4A and FIG. 4B show a first example of a subject interface pad: a patient interface head pad 400 for positioning a patient head. FIG. 5A and FIG. 5B show a second example of a subject interface pad: a patient interface extremity pad 500 for positioning a patient extremity. In one example, the patient interface head pad 400 and the patient interface extremity pad 500 may position a patient body part at an approximately 0° angle. FIG. 6A and FIG. 6B show an example angled positioning pad 600 for increasing a tilt angle of a patient head or extremity, relative to horizontal. In one example, the angled positioning pad may provide a tilt angle of greater than 0°, such as 5°, 10°, or 15°. In another example, the patient interface head pad 400, the patient interface extremity pad 500, and the angled positioning pad 600 may be color coded for ease of use. For example, the patient interface head pad 400 may be green, the patient interface extremity pad 500 may be blue, and the angled positioning pad 600 may be red.

FIG. 4A illustrates a front perspective view of the patient interface head pad 400. In one example, the patient interface head pad 400 may be positioned in a head holder of an imaging device, such as the cradle interface pad 116 for CT imaging system 100 illustrated with respect to FIG. 1. As another example, the pad may be positioned in the cradle interface pad 300, illustrated with respect to FIGS. 3A-3C. In yet other examples, the patient interface head pad 400 may be positioned directly on a patient table, such as the table 114 of FIGS. 1-2. In one example, the patient interface head pad 400 provides a 0° angle for a patient head to rest during a procedure. For example, when positioned in the cradle interface pad 300 or on the table 114, the patient head positioned in the patient interface head pad 400 may be in line with the patient's body. The patient interface head pad 400 comprises a central axis 499, which lies in an x-z plane.

The patient interface head pad 400 comprises a head cradle or cradle 402, which may receive a head of a patient. The cradle 402 includes a body 404 with a first side 406 and a second side 408 extending therefrom. The body 404 comprises a substantially planar surface or planar surface 410 against which the patient's head may rest. The first side 406 and the second side 408 may extend from opposite edges of the body 404. The patient interface head pad 400 includes a first end 412 and a second end 414. When positioned in a CT system, such as positioned in the concave recess of the cradle interface pad 116 of FIG. 1, the second end 414 faces the gantry 102 and the first end 412 faces away from the gantry 102.

In one example, dimensions of the patient interface head pad 400 include a first dimension 422 that is greater than a second dimension 424. In other words, the sides 406, 408 of the cradle 402 include a wider, first region 428 nearer the body 404 and a narrower, second region 430 as the sides extends upward along the y-axis. The wider, first region 428 forms a curve joining the second region 430 of the sides 406, 408 and the body 404. A third dimension 426 of the cradle 402, e.g., a cradle height, may be less than the first dimension 422, e.g., a cradle length. The cradle 402 is formed with a wall 439 having a material thickness 432. The material thickness 432 of the wall 439 may be approximately the same throughout the patient interface head pad 400. The patient interface head pad 400 includes a width 435 of the body 404. In one example, the width 435 of the body 404 may be similar to the second dimension 424 of the sides 406, 408.

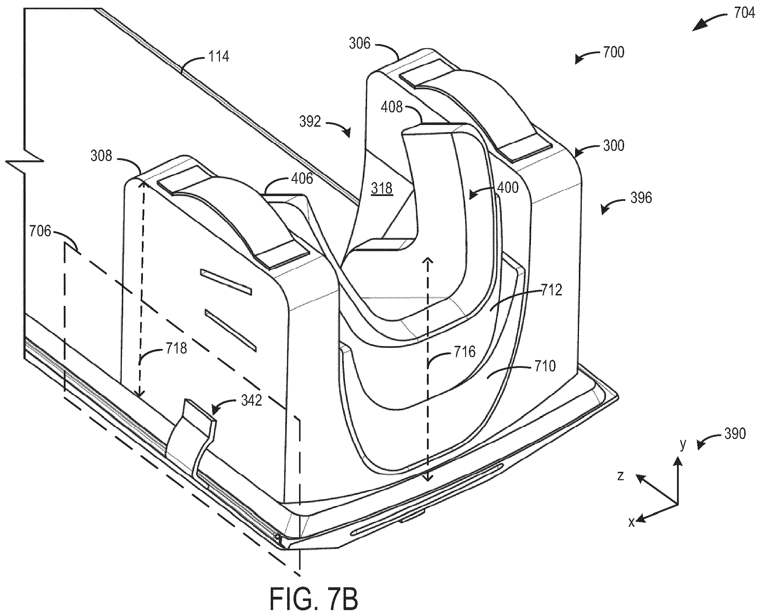

The first end 412 of the cradle includes a first opening 434. The first opening 434 is formed by a cutaway of the wall 439 between the wider, first region 428 and the narrower, second region 430 of the sides 406, 408. In some use cases, the first opening 434 may be approximately flush with the first end 392 or the second end 396 of the concave recess 302 of the cradle interface pad 300. A second opening 436 on the second end 414 of the cradle 402 may be perpendicular with respect to the planar surface of the body 404. In some use cases, the second opening 436 may be approximately flush with the first end 392 or the second end 396 of the concave recess 302 of the cradle interface pad 300, as further described with respect to FIGS. 7B, 9A, and 10B. The patient interface head pad 400 includes an exterior surface 420. In one example, the exterior surface 420 is an outward facing surface of the first side 406, the body 404, and the second side 408. When positioned in the cradle interface pad 300, the exterior surface 420 makes face-sharing contact with the interior surface of the concave recess 302 (e.g., the first side 310 of the base 304, the first interior side 318 of the first wall 306 and the second interior side 320 of the second wall 308), as further described with respect to FIGS. 10A and 10B. An interior surface of the cradle comprises the planar surface 410 and side surface 418. A perimeter surface 438 joins the interior surface and the exterior surface 420. In one example, the perimeter surface 438 may meet the exterior surface 420 at an acute angle.

FIG. 4B illustrates an underside view 450 of the patient interface head pad 400. Particularly, the underside view 450 shows the patient interface head pad 400 viewed from the second end 414. The underside view 450 shows a portion of the side surface 418, the exterior surface 420, and the perimeter surface 438 therebetween. In one example, the second opening 436 may be u-shaped.

A recess 452 formed in a body underside 456 of the patient interface head pad 400 may provide a depression wherein a fastening material 454 may be placed. In one example, the recess 452 may be positioned adjacent to the second end 414 of the cradle 402. In one example, the fastening material 454 may be a matching fastener for mating with the receiving material 386 positioned in the concave recess of the cradle interface pad 300 or a receiving material positioned in the cradle interior of an angled positioning pad (e.g., see FIG. 6A and FIG. 6B below). For example, the fastening material 454 may make face sharing contact with receiving material 386 for securing the patient interface head pad 400 to the cradle interface pad 300. In one example, the fastening material 454 may be made of a hook and loop fastener or a similar fastening material which is complementary to the receiving material 386.

FIG. 5A illustrates a front perspective view of the patient interface extremity pad 500. In one example, the patient interface extremity pad 500 may be positioned in a head holder of an imaging device, such as the cradle interface pad 116 for CT imaging system 100 illustrated with respect to FIG. 1. As another example, the patient interface extremity pad 500 may be positioned in the concave recess of the cradle interface pad 300 illustrated with respect to FIGS. 3A-3C. In yet other examples, the patient interface extremity pad 500 may be positioned directly on a patient table, such as the table 114 of FIG. 1. In one example, the patient interface extremity pad 500 provides a 0° angle for a patient head to rest during a procedure. For example, when positioned in the cradle interface pad 300 or on the table 114, the patient extremity positioned in the patient interface extremity pad 500 may be in line with the patient's body. The patient interface extremity pad 500 comprises a central axis 599, which lies in an x-z plane.

The patient interface extremity pad 500 comprises an extremity cradle or cradle 502, which may receive a patient extremity. The cradle 502 includes a body 504 with a first side 506 and a second side 508 extending therefrom. The body 504 comprises a substantially planar surface or planar surface 510 against which the patient's extremity may rest. The first side 506 and the second side 508 may extend from opposite edges of the body 504. The patient interface extremity pad 500 includes a first end 512 and a second end 514. When positioned in a CT system, such seated against the cradle interface pad 116 in FIG. 1, the patient interface extremity pad 500 may be configured in a first position or a second position. For example, when configured in the first position, the first end 512 may face the gantry 102 and when configured in the second position the first end 392 may face away from the gantry 102. Further detail describing configurations of the patient interface extremity pad 500 are described with respect to FIGS. 8A and 8B.

**In** one example, along the z-axis, the sides 506, 508 of the patient interface extremity pad are approximately trapezoidal. For example, the second side 508, and the first side 506 (e.g., which mirrors the second side 508), is formed with a first base 542 of a first dimension 522 that is approximately parallel with second base 544 of a second dimension 524. The first dimension 522 is more than twice magnitude of the second dimension 524. The second side 508 includes a first side 546 of a third dimension 526. The first side 546 is arranged approximately perpendicular to the first base 542 and the second base 544. The second side 508 of the patient interface extremity pad 500 includes a second side 548 of a fourth dimension 550. The second side 548 is arranged at an acute angle with respect to the first base 542 and an obtuse angle with respect to the second base 544. The sides 506, 508 of the cradle 502 include a first region 528 nearer to the body 504 and a second region 530 further from the body 504 along the y-axis. In one example, a width 532 of the body 504 may be greater than the second dimension 524 and less than the first dimension 522 of the sides 506, 508.

The patient interface extremity pad 500 includes the planar surface 510 of the body 504, a second interior surface 518 in the first region 528 of the sides 506, 508, a third interior surface 552 in the second region 530 of the sides 506, 508, and an exterior surface 520. In one example, the exterior surface 520 is an outward facing surface of the first side 506, the body 504, and the second side 508. The exterior surface 520 of the patient interface extremity pad 500 may be u-shaped. In one example, when positioned in the cradle interface pad 300, the exterior surface 520 may make face sharing contact with the interior surface of the concave recess 302 (e.g., the first side 310 of the base 304, the first interior side 318 of the first wall 306 and the second interior side 320 of the second wall 308). A perimeter surface 538 joins the interior surfaces 510, 518, 552 and the exterior surface 520.

The first end 512 of the cradle includes a first opening 534. The first opening 534 comprises a portion of the perimeter surface 538, including a ridge 554, first angled surface 556, and second angled surface 558. In some examples, the first opening 534 may be approximately flush with the first end 392 or the second end 396 of the concave recess 302 of the cradle interface pad 300, as further described with respect to FIGS. 8A-8B. A second opening 536 on the second end 514 of the cradle 502 may be approximately perpendicular with respect to the planar surface 510 of the body 504. In some examples, the second opening 536 may be approximately flush with the first end 392 or the second end 396 of the concave recess 302 of the cradle interface pad.

FIG. 5B illustrates an underside view 560 of the patient interface extremity pad 500. The underside view 560 shows the second opening 536 of the patient interface extremity pad 500 as seen from the second end 514. The underside view 560 shows the second interior surface 518, the third interior surface 552, the exterior surface 520, and the perimeter surface 538 therebetween.

The patient interface extremity pad 500 is formed with a wall 561 having an irregular material thickness. For example, the wall 561 may be formed to have a first thickness 562 in the first region 528 of the pad. The wall 561 may increase to a second thickness 566 in the second region 530 of the sides 506, 508. In other examples, the patient interface extremity pad may be formed with a wall having a material thickness approximately the same throughout.

The second opening 536 has a u-shaped, exterior edge 570 and an irregular, interior edge 572. The interior edge 572 of the second opening 536 is narrower near the body 504, wider towards an upper portion 568 of the sides 506, 508, and narrowest at a transition 584 between the first region 528 and the second region 530 of the sides 506, 508. For example, the interior edge 572 may be the width 532 near the body 504, narrowing to a second width 574 at the transition 584, and increasing to third width 576 towards the upper 568 of the sides 506, 508.

A recess 578 formed in the in the body underside 580 may provide a depression wherein a fastening material 582 may be placed. In one example, the recess 578 may be positioned adjacent to the second end 514 of the cradle 502. In one example, the fastening material 582 may be a matching fastener for mating with receiving material positioned in the concave recess 302 of the cradle interface pad 300 (e.g., see FIG. 3C) or positioned in the cradle interior of an angled positioning pad (e.g., see FIG. 6A and FIG. 6B below). For example, the fastening material 582 may make face sharing contact with receiving material 386 for securing the patient interface head pad to the cradle interface pad 300. The fastening material 582 may be made from a hook and loop fastener or similar fastening material which is complementary to the receiving material 386.

FIG. 6A illustrates a front perspective view of the angled positioning pad 600. In one example, the angled positioning pad 600 may be positioned in a head holder of an imaging device, such as the cradle interface pad 116 for CT imaging system 100 illustrated with respect to FIG. 1. As another example, the angled positioning pad 600 may be positioned in the concave recess of the cradle interface pad 300 illustrated with respect to FIGS. 3A-3C. In yet other examples, angled positioning pad 600 may be positioned directly on a patient table, such as the table 114 of FIG. 1. In one example, the angled positioning pad 600 provides a greater than 0° tilt angle 652 (in FIG. 6B) for a patient head or extremity to rest during a procedure. For example, when positioned in the cradle interface pad 300 or on the table 114, the angled positioning pad may angle a patient head or extremity at a positive or negative angle, with respect to the patient's body. The angled positioning pad 600 comprises a central axis 699, which lies in an x-z plane.

The angled positioning pad 600 comprises a cradle 602, which may receive a patient head or extremity, in some examples. Additionally or alternatively, the angled positioning pad 600 may receive one or more of a patient interface head pad, a patient interface extremity pad, or a second angled positioning pad, such as described in further detail with respect to FIGS. 7A-10B. The cradle 602 includes a body 604 with a first side 606 and a second side 608 extending therefrom. The first side 606 and the second side 608 may extend from opposite edges of the body 604. The body 604 comprises an interior surface 610 against which the patient or the one or more additional pads may rest. The body 604 further comprises an exterior surface 620. In one example, the exterior surface 620 is an outward facing surface of the first side 606, the body 604, and the second side 608. A perimeter surface 638 joins the interior surface 610 and the exterior surface 620.

The angled positioning pad 600 includes a first end 612 and a second end 614. When positioned in a CT system, such seated in the cradle interface pad 116 of FIG. 1, the angled positioning pad 600 may be configured in a first position or a second position. For example, when configured in the first position, the first end 612 may face the gantry 102 and when configured in the second position the first end 612 may face away from the gantry 102. Further configurations of the angled positioning pad 600 in the subject positioning kit are described with respect to FIGS. 7A-10B. The first end 612 of the angled positioning pad 600 includes a first opening 634. In some examples, the first opening 634 may be approximately flush with the first end 392 or the second end 396 of the concave recess 302 of the cradle interface pad 300, as described with respect to FIGS. 7A, 8B, and 9B. A second opening 636 on the second end 614 of the angled positioning pad 600 pad may be arranged approximately perpendicular with respect to a base 641 of the body 604. In some examples, the second opening 636 may be approximately flush with the first end 392 or the second end 396 of the concave recess 302, as described with respect to FIGS. 7B, 8A, and 9A.

The angled positioning pad 600 is formed with wall 644 having variable material thickness throughout. For example, the thickness of the wall 644 may increase from the first end 612 towards the second end 614. The thickness of the wall 644 may decrease from an upper portion 646 towards a lower portion 648 of the sides 608, 608.

A first recess 640 is formed in the in body 604 and may provide a depression wherein a receiving material 642 may be placed. In one example, the first recess 640 may be positioned adjacent to the second end 614 of the cradle 602. In one example, the receiving material 642 may be a matching fastener for mating with fastening material (e.g., fastening material 454) of the patient interface head pad 400 and the patient interface extremity pad 500 (e.g., fastening material 582). For example, the receiving material 642 may make face sharing contact with fastening material 454 for securing the patient interface head pad 400 to angled positioning pad 600. The receiving material 642 may be made of a hook and loop fastener or similar receiving material which is complementary to fastening material, such as the fastening material 454 of the patient interface head pad 400 and/or the fastening material 582 of the patient interface extremity pad 500.

FIG. 6B illustrates cross section 650 of the angled positioning pad 600. Particularly, the cross section 650 shows an example tilt angle 652 of the body 604 and example fasteners. The cross section 650 shows the increasing thickness, e.g., at tilt angle 652, of the wall 644 from the first end 612 to the second end 614. The tilt angle 652 may provide a greater than 0° angle for a patient head or extremity to rest during a procedure. In one example, the tilt angle may be 15°.

In one example, looking down the z-axis, the angled positioning pad 600 is approximately trapezoidal. For example, the second side 608 is formed with a first base 654 of a first dimension 662 that is approximately parallel with a second base 656 of a second dimension 664. The first dimension 662 is approximately twice magnitude of the second dimension 664. The second side 608 includes a first side 658 of a third dimension 666. The first side 658 is arranged approximately perpendicular to the first base 654 and the second base 656. The second side 608 of the angled positioning pad 600 includes a second side 660 of fourth dimension 668. The second side 660 is arranged at an acute angle with respect to the first base 654 and an obtuse angle with respect to the second base 656. The second side 608 further includes a rectangular portion 670 having a height of a fifth dimension 672 and a length of the second dimension 664. The rectangular portion 670 may be continuous with a lower portion 674 of the wall 644.

The cross section 650 shows the depression formed by first recess 640 and the receiving material 642 placed therein. Similarly, a second recess 676 may form a depression in the first base 654 where a fastening material 643 may be positioned. In one example, the first recess 640 and the second recess 676 may be positioned adjacent to the second end 614 of the pad, the fastening material 643 mating with and securing to receiving material 386 positioned in the concave recess 302 of the cradle interface pad 300 (e.g., as further described with respect to FIGS. 7A-10B). In one example, the fastening material 643 may be made from a hook and loop fastener or similar fastening material which is complementary to the receiving material 386.

As described above, the subject positioning kit may include the cradle interface pad 300, the patient interface head pad 400, the patient interface extremity pad 500, and at least one angled positioning pad 600. The subject positioning kit may be configurable to have multiple combinations of angled positioning pads and patient interface pads positioned in the concave recess 302 of the cradle interface pad 300, depending on a desired imaging anatomy (e.g., head or extremity) and angle at which to position the anatomy.

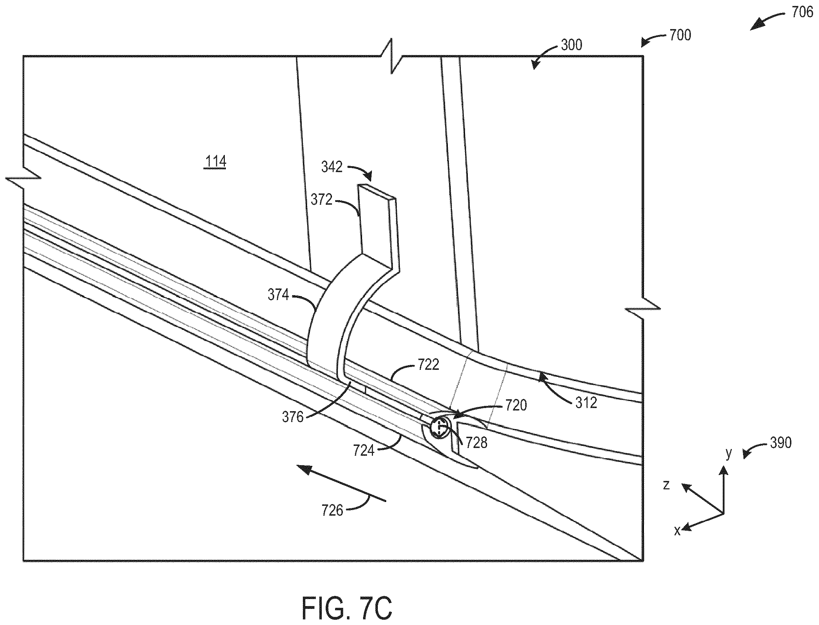

FIGS. 7A-7C show a first configuration 700 of the subject positioning kit where the cradle interface pad 300 is coupled to a table (e.g., the table 114 of the CT imaging system 100) via the at least one attachment points, and the cradle interface pad 300 further has two angled positioning pads 600 and the patient interface head pad 400 stacked in the concave recess 302 to create a positive angle. A first view 702 is shown in FIG. 7A, a second view 704 is shown in FIG. 7B, and a third view 706 is shown in FIG. 7C. Each of a first angled positioning pad 710 and a second angled positioning pad 712 are configured as described with respect to the angled positioning pad 600.

In the first configuration 700, the base 641 of the body 604 of the first angled positioning pad 710 is positioning in face-sharing contact with the first side 310 of the base 304 of the cradle interface pad 300. In some embodiments where the cradle interface pad 300 is configured with a receiving material, as described with respect to FIG. 3C, the fastening material 643 of the first angled positioning pad 710 may couple with the receiving material of the cradle interface pad 300 to secure the first angled positioning pad 710 in the concave recess 302. Further, the exterior surface 620 of the first side 606 and the second side 608 of the first angled positioning pad 710 may be in face-sharing contact with the second interior side 320 of the second wall 308 and the first interior side 318 of the first wall 306 of the cradle interface pad 300, respectively.

The base 641 of the body 604 of the second angled positioning pad 712 is positioned in face-sharing contact with the interior surface 610 of the first angled positioning pad 710. The exterior surface 620 of the second angled positioning pad 712, including the exteriors surface of the first side 606 and the second side 608 of the second angled positioning pad 712 may be in face-sharing contact with the interior surface 610 of the first angled positioning pad 710. The fastening material 643 of the second angled positioning pad 712 may be coupled to the receiving material 642 of the first angled positioning pad 710 to couple the first angled positioning pad 710 to the second angled positioning pad 712.

The first end 612 of each of the first angled positioning pad 710 and the second angled positioning pad 712 may be positioned facing the same direction as the first end 392 of the cradle interface pad 300. The first end 392 may face away from a gantry and towards a length of a table of an imaging system, such as the gantry 102 and the table 114 of the CT imaging system 100, described with respect to FIG. 1. In this way, the first angled positioning pad 710 and the second angled positioning pad 712 may collectively provide a positive angle 714, relative to the first side 310 of the base 304 of the cradle interface pad 300, which is substantially planar, at which a patient head may be positioned. For example, when the tilt angle 652 of each of the first angled positioning pad 710 and the second angled positioning pad 712 is 15 degrees, combination of the first angled positioning pad 710 and the second angled positioning pad 712 may provide the positive angle 714, equal to 30 degrees.

The patient interface head pad 400 is shown in the first configuration 700 as positioned on top of the second angled positioning pad 712. In other configurations, the patient interface head pad 400 may be replaced with the patient interface extremity pad 500. As shown in FIG. 7A, the body underside 456 of the patient interface head pad 400 is positioned in face-sharing contact with the interior surface 610 of the second angled positioning pad 712. The exterior surface 420 of the patient interface head pad 400, including the exteriors surface of the first side 406 and the second side 408 of the patient interface head pad 400 may be in face-sharing contact with the interior surface 610 of the second angled positioning pad 712. The fastening material 454 of the patient interface head pad 400 may be coupled to the receiving material 642 of the second angled positioning pad 712 to couple the patient interface head pad 400 to the second angled positioning pad 712. As the body 404 of the patient interface head pad 400 is not angled (e.g., comprises a substantially planar or planar surface 410), positioning of the patient interface head pad 400 on the second angled positioning pad 712 may not increase a tilt angle (e.g., the positive angle 714) at which a patient head may be positioned.

As shown in FIG. 7B, each of the first angled positioning pad 710, the second angled positioning pad 712, and the patient interface head pad 400 are vertically aligned 716 with the second end 396 of the cradle interface pad 300. In the first configuration 700, a height of the first side 406 and the second side 408 of the patient interface head pad 400 may extend above a height 718 of the cradle interface pad 300. In other configurations, as described herein, the height of the patient interface head pad 400 may not extend above the height 718 of the cradle interface pad 300.

As described with respect to FIG. 3C, each of the at least one attachment points mounted on each of the first wall 306 and the second wall 308 are configured to interface with a track of a table. The table may be configured with a track on a first side of the table, as shown in FIG. 7C, and on a second side of the table, opposite the first side. Attachment of the cradle interface pad 300 to a table by inserting at least one attachment point into a track of the table is herein described with respect to a track on a first side of the table, although it is to be understood that the description herein may also be applied to the second side of the table.

FIG. 7C shows a track 720 of a table in detail, where the table may be an example of the table 114 of the CT imaging system 100 of FIG. 1. The track 720 is configured as having a C shape, such that a top 722 and a bottom 724 of the track 720 form an interior diameter into which the first attachment point 342 of the cradle interface pad 300 may be positioned. A gap between the top 722 and the bottom 724 of the track 720 enables the hook end 376 of the attachment point to be slid into the track 720, as further described with respect to FIG. 7C.

The hook end 376 may be slid into the track 720 of the table 114 in a direction indicated by an arrow 726. As described with respect to FIG. 3C, the hook end 376 is configured with an end diameter which is greater than a width of the curved extension 374 and the mounting section 372. The end diameter (not shown in FIG. 7C) may be less than a diameter 728 of the track 720. This enables the hook end 376 to be slid into the track 720 of the table 114 in the direction indicated by the arrow 726 and be retained in the track 720, thus not sliding out of the track 720 in a lateral direction (e.g., along the x-axis, with respect to the coordinate system 390). Hook end 376 is thus positioned between the top 722 and the bottom 724 of the track 720. More specifically, the hook end 376 may be partially surrounded by the track 720. A portion of the hook end 376 may be exposed through and extend through the gap of the track 720, allowing the hook end 376 to slide along a length of the track 720.

An angle of curvature of the second side 312 of the base 304 of the cradle interface pad 300 is complementary to an angle of curvature of the table 114. This enables the cradle interface pad 300 to be positioned in face-sharing contact with the table 114 such that no additional padding or interface is positioned therebetween. This may reduce undesired bumps, curves, or other profiles which may deform the base 304 and, consequently, the concave recess 302. Thus, the patient head and/or extremity positioned in the patient interface head pad 400 and/or the patient interface extremity pad 500, respectively, may be positioned at a desired angle for patient imaging.

A second configuration 800 of the subject positioning kit is shown in FIGS. 8A and 8B. The second configuration 800 is similar to the first configuration 700 and includes a first angled positioning pad 810 and a second angled positioning pad 812, each of which are configured as the angled positioning pad 600 of FIGS. 6A-6B, and a patient interface extremity pad 500 stacked in the concave recess 302 of the cradle interface pad 300. The first angled positioning pad 810, the second angled positioning pad 812, and the patient interface extremity pad 500 are positioned to create a negative angle at which a patient extremity may be positioned for imaging.

As described with respect to FIGS. 7A-7C, stacking two angled positioning pads configured as the angled positioning pad 600 creates a 30-degree angle with respect to the first side 310 of the base 304 of the cradle interface pad 300 (e.g., which is substantially planar), which may allow the patient extremity to be positioned at the 30-degree angle. As shown in FIG. 8A, the second end 614 of each of the first angled positioning pad 810 and the second angled positioning pad 812 may be positioned facing the same direction as and vertically aligned 816 with the first end 392 of the cradle interface pad 300. The second end 396 of the cradle interface pad 300 may face towards a gantry and away from a length of a table of an imaging system, such as the gantry 102 and the table 114 of the CT imaging system 100, described with respect to FIG. 1. Described another way, the first angled positioning pad 810, the second angled positioning pad 812, and the patient interface extremity pad 500 may be positioned in the concave recess 302 in a position which is 180-degree rotated compared to positioning of the first angled positioning pad 710, the second angled positioning pad 712, and the patient interface head pad 400 in the first configuration 700. In this way, positioning of the second configuration 800, including the first angled positioning pad 810 and the second angled positioning pad 812, may collectively provide a negative angle 814, relative to the first side 310 of the base 304 of the cradle interface pad 300 (e.g., which is substantially planar). For example, when the tilt angle 652 of each of the first angled positioning pad 810 and the second angled positioning pad 812 is 15 degrees, combination of the first angled positioning pad 810 and the second angled positioning pad 812 may provide a 30-degree angle. Positioning the cradle interface pad 300 with the second end 396 facing the gantry and facing away from the length of the table 114.

In some examples, a coupling strap 820 may be used to couple each of the first angled positioning pad 810, the second angled positioning pad 812, and the patient interface extremity pad 500 to each other and to the cradle interface pad 300. The coupling strap 820 may be included in the subjecting positioning kit and may be used in other configurations thereof, such as configurations described with respect to FIGS. 7A-7C, FIGS. 9A-9B, and FIGS. 10A-10B. The coupling strap may be similar to the positioning strap 336 and may include a receiving portion on a first side 826 of the coupling strap 820 at both a first strap end 822 and a second strap end 824, opposite the first strap end 822. The coupling strap 820 may further include a fastening portion on a second side of the coupling strap 820, opposite the first side 826, at both the first strap end 822 and the second strap end 824. For example, the receiving portion of the coupling strap 820 may be a receiving material which is a contrasting material with respect to the fastening material of the fastening portion. The receiving material may further be contrasting, complementary material to fastening material 643 of the angled positioning pad 600, fastening material 454 of the patient interface head pad 400, and fastening material 582 of the patient interface extremity pad 500. The fastening material may further be contrasting, complementary material to receiving material 386 of the cradle interface pad 300 and receiving material 642 of the angled positioning pad 600. As shown in FIG. 8A, this may enable the first strap end 822 of the coupling strap 820 to be positioned between the patient interface extremity pad 500 and the second angled positioning pad 812, such that the receiving portion is coupled to the fastening material 582 of the patient interface extremity pad 500 and the fastening portion is coupled to the receiving material 642 of the angled positioning pad 600. The second strap end 824 of the coupling strap 820 may be positioned between the first angled positioning pad 810 and the cradle interface pad 300, such that the receiving portion is coupled to the fastening material 643 of the first angled positioning pad 810 and the fastening portion is coupled to the receiving material 386 of the cradle interface pad 300.

A weight of a patient's extremity positioned in the patient interface extremity pad 500 may compress the respective fastening materials and receiving materials such that undesired bumps, curves, or other profiles which may deform the angle created by the first angled positioning pad 810 and the second angled positioning pad 812 are not present. Thus, the patient extremity positioned in the patient interface extremity pad 500, may be positioned at a desired angle for patient imaging and the angle of the patient's extremity may not change during imaging due to movement of the first angled positioning pad 810, the second angled positioning pad 812, and/or the patient interface extremity pad 500.

A third configuration 900 of the subject positioning kit shown in FIGS. 9A and 9B includes one angled positioning pad 910, configured as the angled positioning pad 600, and the patient interface head pad 400 stacked in the concave recess 302 of the cradle interface pad 300. A rear view 920 of the third configuration 900 is shown in FIG. 9B. The angled positioning pad 910 is coupled to the cradle interface pad 300 and to the patient interface head pad 400 via the fastening material 643 and the receiving material 642, respectively, as described with respect to FIGS. 7A-8B. The angled positioning pad 910 creates an angle of 15-degrees for the patient interface head pad 400. The second end 396 of the cradle interface pad 300 may face towards a gantry and away from a length of a table of an imaging system, such as the gantry 102 and the table 114 of the CT imaging system 100, described with respect to FIG. 1. In this way, the angled positioning pad 910 may provide a positive angle 914, relative to the first side 310 of the base 304 of the cradle interface pad 300, at which a patient head may be positioned. For example, when the tilt angle 652 of the angled positioning pad 910 is 15 degrees, positioning of the patient interface head pad 400 on top of the angled positioning pad 910 may provide a patient head tilt angle of 15-degrees. As further shown in FIGS. 9A and 9B, the positioning strap 336 may be threaded through the first slot 326 of the first wall 306 and the third slot 330 of the second wall 308.

A fourth configuration 1000 of the subject positioning kit shown in FIGS. 10A and 10B includes the patient interface head pad 400 positioned in the concave recess 302 of the cradle interface pad 300, creating a negligible angle. A rear view 1020 of the fourth configuration 1000 is shown in FIG. 10B. The patient interface head pad 400 may be coupled to the cradle interface pad 300 by mating the fastening material 454 of the patient interface head pad 400 with the receiving material 386 of the cradle interface pad 300. Thus, the body underside 456 of the patient interface head pad 400 is in face-sharing contact with the concave recess 302 of the cradle interface pad 300. The fourth configuration 1000 may be used when it is desirable for a patient head to lay approximately flat during imaging.

FIGS. 7A-10B show multiple potential configurations of the subject positioning kit, non-limiting, other combinations of angled pads and interface pads possible. In each of the configurations, the positioning strap 336 as described with respect to FIGS. 3A-3C may be used to assist in positioning a patient's head and/or extremity in the respective patient interface head pad 400 or patient interface extremity pad 500. Slots which the positioning strap 336 passes through (e.g., the first slot 326 and the third slot 330, the second slot 328 and the fourth slot 332, or another pair of slots with one slot on the first wall 306 and another slot on the second wall 308), as well as which fastening panel on each of the first wall 306 and the second wall 308 the positioning strap 336 is coupled to may depend on a size of the patient head and/or extremity, a number of angled positioning pads 600 stacked in the concave recess 302 of the cradle interface pad 300, and which of the patient interface head pad 400 and the patient interface extremity pad 500 is used. Further, the coupling strap 820 may be used as described with respect to FIGS. 8A and 8B in any of the above described or other potential configurations without departing from the scope of the present disclosure.

In one aspect, a cradle interface pad may position and support a patient head or extremity and provide a range of discrete angles when used in combination with one or more pads. The combination of the pads, color coding, and the cradle interface pad may allow quick, precise, and optimal positioning of a patient head or extremity. Further, the at least one attachment point of the cradle interface pad may allow simple and seamless positioning of the cradle interface pad on a table of an imaging system and removal from the table, where a patient head and/or extremity may be secured in the cradle interface pad during positioning of the cradle interface pad on the table. The technical effect of using the cradle interface pad, patient interface pads, and optional angled positioning pads is to allow repeatable scans of a target area while allowing an operator to easily and stably adjust the tilt of the scanned subject with minimal patient disturbance and reduced incidence of pinching. By doing this, a quality of medical imaging may be enhanced.

The present disclosure also wherein a first end of a strap is coupled to the first wall via the coupling material and a second end of the strap is coupled to the second wall via the coupling material. Further, a first end of the coupling strap is coupled to a bottom of the cradle interface pad and a fastening material of the top angled positioning pad or subject interface pad.

The disclosure also provides support for a cradle interface pad for use with an imaging system, the cradle interface pad comprising: a base with a planar first side and a curved second side, opposite the first side, a first wall positioned on a first end of the base and a second wall positioned on a second end of the base, wherein the first wall, the second wall, and the planar first side of the base form a concave recess of the cradle interface pad, at least one slot extending through each of the first wall and the second wall, at least one attachment point mounted on each of the first wall and the second wall and extending towards the base, and a handle positioned on a top face of each of the first wall and the second wall. In a first example of the system, the at least one slot includes a first slot and a second slot, the first slot positioned above the second slot. In a second example of the system, optionally including the first example, the second slot is parallel to a top edge of the first wall and the first slot is angled at a non-zero angle, relative to the top edge of the first wall. In a third example of the system, optionally including one or both of the first and second examples, the system further comprises: a positioning strap extending through each of the first slot on the first wall and the second wall and/or the positioning strap extending through each of the second slot on the first wall and the second wall. In a fourth example of the system, optionally including one or more or each of the first through third examples, each of the at least one attachment point include a hook end configured to interface with a track of a table. In a fifth example of the system, optionally including one or more or each of the first through fourth examples, the cradle interface pad is configured with a fastening material which extends along a length of each of the first wall and the second wall. In a sixth example of the system, optionally including one or more or each of the first through fifth examples, the concave recess is configured to have a subject interface pad positioned therein, such that a body underside of the subject interface pad is in face-sharing contact with the concave recess. In a seventh example of the system, optionally including one or more or each of the first through sixth examples, the concave recess is configured to have at least one angled positioning pad positioned therein, such that a base of a body of the angled positioning pad is in face-sharing contact with the planar first side of the base of the cradle interface pad. In an eighth example of the system, optionally including one or more or each of the first through seventh examples, an interior surface of the angled positioning pad is configured to be in face-sharing contact with a body underside a subject interface pad and/or the base of the body of a second angled positioning pad, the second angled positioning pad having the same configuration as the angled positioning pad. In a ninth example of the system, optionally including one or more or each of the first through eighth examples, each of the at least one angled positioning pad and the subject interface pad are coupled using a coupling strap and/or fasteners and receivers therebetween.

The disclosure also provides support for a subject positioning kit, comprising: a cradle interface pad having a concave recess, at least one angled positioning pad configured to be positioned in the concave recess of the cradle interface pad, and a subject interface pad configured to be positioned in the concave recess of the cradle interface pad and further configured to be positioned in face-sharing contact with an interior surface of the at least one angled positioning pad. In a first example of the system in a first configuration: a body underside of the subject interface pad is configured to be positioned in face-sharing contact with the interior of a first angled positioning pad of the at least one angled positioning pad, and a base of a body of the first angled positioning pad is configured to be positioned in face-sharing contact with the interior of a second angled positioning pad of the at least one angled positioning pad and/or to be positioned in face-sharing contact with the concave recess of the cradle interface pad. In a second example of the system, optionally including the first example when the base of the body of the first angled positioning pad is in face-sharing contact with the interior of the second angled positioning pad, the base of the body of the second angled positioning pad is in face-sharing contact with the concave recess of the cradle interface pad. In a third example of the system, optionally including one or both of the first and second examples, the subject interface pad is configured with a fastening material on a body underside of the subject interface pad and each of the concave recess of the cradle interface pad and an interior of the at least one angled positioning pad are configured with a receiving material which is complementary to the fastening material of the subject interface pad, such that the subject interface pad is coupled to the concave recess of the cradle interface pad and/or the interior of the at least one angled positioning pad by mating the fastening material with the receiving material. In a fourth example of the system, optionally including one or more or each of the first through third examples, the at least one angled positioning pad is further configured with the fastening material on the base of the body of the angled positioning pad, such that, the angled positioning pad is coupled to the concave recess of the cradle interface pad by mating the fastening material of the angled positioning pad with the receiving material of the cradle interface pad and, the angled positioning pad is coupled to a second angled positioning pad, having the same configuration as the angled positioning pad, by mating the fastening material of the angled positioning pad with the receiving material of the second angled positioning pad. In a fifth example of the system, optionally including one or more or each of the first through fourth examples in a second configuration, a first end of the at least one angled positioning pad is positioned facing the same direction as a first end of the cradle interface pad to create a positive angle and, in a third configuration, the first end of the at least one angled positioning pad is facing the same direction as a second end of the cradle interface pad, opposite the first end, to create a negative angle.

The disclosure also provides support for a system, comprising: a patient table, and a cradle interface pad selectively coupled to the patient table, where the cradle interface pad comprises: a concave recess formed by a base, a first wall, and a second wall, opposite the first wall, and at least one attachment point mounted on each of the first wall and the second wall and extending towards the base. In a first example of the system, each of the at least one attachment point is formed as a mounting section, the mounting section coupling the at least one attachment point to the first wall or the second wall, a curved extension coupled to the mounting section, and a hook end coupled to the curved extension, the hook end having an end diameter. In a second example of the system, optionally including the first example, the patient table is configured with a track having a top and a bottom which form a C shape with an interior diameter, the interior diameter being greater than the end diameter of the hook end. In a third example of the system, optionally including one or both of the first and second examples, the cradle interface pad is coupled to the patient table when the hook end is positioned in the interior diameter of the track between the top and the bottom of the track.

As used herein, an element or step recited in the singular and preceded with the word "a" or "an" should be understood as not excluding plural of said elements or steps, unless such exclusion is explicitly stated. Furthermore, references to "one embodiment" of the invention do not exclude the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art.

## Claims

1. A cradle interface pad (300) for use with an imaging system, the cradle interface pad comprising:
a base (304) with a planar first side (310) and a curved second side (312), opposite the planar first side;
a first wall (306) positioned on a first end of the base and a second wall (308) positioned on a second end of the base, wherein the first wall, the second wall, and the planar first side of the base form a concave recess of the cradle interface pad;
at least one slot (326, 328, 330, 332) extending through each of the first wall and the second wall;
at least one attachment point (342, 352) mounted on each of the first wall and the second wall and extending towards the base; and
a handle (344, 346) positioned on a top face of each of the first wall and the second wall, wherein the handles are configured to provide an element of the cradle interface pad for a patient to grasp with their hands during imaging to assist in moving the patient's hands, arms, and shoulders away from a region to be imaged.

2. The cradle interface pad of claim 1, wherein the at least one slot includes a first slot and a second slot, the first slot positioned above the second slot.

3. The cradle interface pad of claim 2, wherein the second slot is parallel to a top edge of the first wall and the first slot is angled at a non-zero angle, relative to the top edge of the first wall.

4. The cradle interface pad of claim 2, further comprising a positioning strap extending through each of the first slot on the first wall and the second wall and/or the positioning strap extending through each of the second slot on the first wall and the second wall.

5. The cradle interface pad of claim 1, wherein each of the at least one attachment point include a hook end configured to interface with a track of a table, the attachment point comprising a mounting section (372), a curved extension (374) coupled to the mounting section (372), and a hook end (376) coupled to the curved extension (374).

6. The cradle interface pad of claim 1, wherein the cradle interface pad is configured with a fastening material which extends along a length of each of the first wall and the second wall.

7. The cradle interface pad of claim 1, wherein the concave recess is configured to have a subject interface pad positioned therein, such that a body underside of the subject interface pad is in face-sharing contact with the concave recess.

8. The cradle interface pad of claim 1, wherein the concave recess is configured to have at least one angled positioning pad positioned therein, such that a base of a body of the angled positioning pad is in face-sharing contact with the planar first side of the base of the cradle interface pad.

9. The cradle interface pad of claim 8, wherein each of the at least one angled positioning pad and a subject interface pad are coupled using a coupling strap and/or fasteners and receivers therebetween.

10. A subject positioning kit, comprising:
the cradle interface pad (300) of any preceding claim;
at least one angled positioning pad (600) configured to be positioned in the concave recess of the cradle interface pad; and
a subject interface pad (400, 500) configured to be positioned in the concave recess of the cradle interface pad and further configured to be positioned in face-sharing contact with an interior surface (610) of the at least one angled positioning pad.

11. The subject positioning kit of claim 10, wherein, in a first configuration:
a body underside of the subject interface pad is configured to be positioned in face-sharing contact with the interior surface of a first angled positioning pad of the at least one angled positioning pad; and
a base of a body of the first angled positioning pad is configured to be positioned in face-sharing contact with the interior surface of a second angled positioning pad of the at least one angled positioning pad and/or to be positioned in face-sharing contact with the concave recess of the cradle interface pad.

12. The subject positioning kit of claim 11, wherein, when the base of the body of the first angled positioning pad is in face-sharing contact with the interior surface of the second angled positioning pad, the base of the body of the second angled positioning pad is in face-sharing contact with the concave recess of the cradle interface pad.

13. The subject positioning kit of claim 11, wherein the subject interface pad is configured with a fastening material on the body underside of the subject interface pad and each of the concave recess of the cradle interface pad and an interior of the at least one angled positioning pad are configured with a receiving material which is complementary to the fastening material of the subject interface pad, such that the subject interface pad is coupled to the concave recess of the cradle interface pad and/or the interior of the at least one angled positioning pad by mating the fastening material with the receiving material.

14. The subject positioning kit of claim 13, wherein the at least one angled positioning pad is further configured with the fastening material on the base of the body of the angled positioning pad, such that;
the angled positioning pad is coupled to the concave recess of the cradle interface pad by mating the fastening material of the angled positioning pad with the receiving material of the cradle interface pad and;
the angled positioning pad is coupled to the second angled positioning pad, having the same configuration as the angled positioning pad, by mating the fastening material of the angled positioning pad with the receiving material of the second angled positioning pad.

15. The subject positioning kit of claim 10 wherein, in a second configuration, a first end of the at least one angled positioning pad is positioned facing the same direction as a first end of the cradle interface pad to create a positive angle and, in a third configuration, the first end of the at least one angled positioning pad is facing the same direction as a second end of the cradle interface pad, opposite the first end, to create a negative angle.

## Patentansprüche

1. Auflageschnittstellenkissen (300) zur Verwendung mit einem Bildgebungssystem, wobei das Auflageschnittstellenkissen Folgendes umfasst:
eine Basis (304) mit einer planaren ersten Seite (310) und einer gekrümmten zweiten Seite (312) gegenüber der planaren ersten Seite;
eine erste Wand (306), die an einem ersten Ende der Basis positioniert ist, und eine zweite Wand (308), die an einem zweiten Ende der Basis positioniert ist, wobei die erste Wand, die zweite Wand und die planare erste Seite der Basis eine konkave Vertiefung des Auflageschnittstellenkissens bilden;
mindestens einen Schlitz (326, 328, 330, 332), der sich durch sowohl die erste Wand als auch die zweite Wand erstreckt;
mindestens einen Anbringungspunkt (342, 352), der an sowohl der ersten Wand als auch der zweiten Wand montiert ist und sich in Richtung der Basis erstreckt; und
einen Griff (344, 346), der auf einer oberen Fläche sowohl der ersten Wand als auch der zweiten Wand positioniert ist, wobei die Griffe dazu ausgestaltet sind, ein Element des Auflageschnittstellenkissens bereitzustellen, das ein Patient während einer Bildgebung mit seinen Händen ergreifen kann, um ein Bewegen der Hände, Arme und Schultern des Patienten von einem abzubildenden Bereich weg zu unterstützen.

2. Auflageschnittstellenkissen nach Anspruch 1, wobei der mindestens eine Schlitz einen ersten Schlitz und einen zweiten Schlitz umfasst, wobei der erste Schlitz über dem zweiten Schlitz positioniert ist.

3. Auflageschnittstellenkissen nach Anspruch 2, wobei der zweite Schlitz parallel zu einer Oberkante der ersten Wand ist und der erste Schlitz in einem Winkel ungleich null relativ zur Oberkante der ersten Wand abgewinkelt ist.

4. Auflageschnittstellenkissen nach Anspruch 2, ferner umfassend einen Positionierungsgurt, der sich durch jeweils den ersten Schlitz an der ersten Wand und der zweiten Wand erstreckt, und/oder wobei sich der Positionierungsgurt durch jeweils den zweiten Schlitz an der ersten Wand und der zweiten Wand erstreckt.

5. Auflageschnittstellenkissen nach Anspruch 1, wobei jeder des mindestens einen Anbringungspunkts ein Hakenende umfasst, das dazu ausgestaltet ist, mit einer Schiene eines Tisches zu interagieren, wobei der Anbringungspunkt einen Montageabschnitt (372), eine mit dem Montageabschnitt (372) gekoppelte gekrümmte Verlängerung (374) und ein mit der gekrümmten Verlängerung (374) gekoppeltes Hakenende (376) umfasst.

6. Auflageschnittstellenkissen nach Anspruch 1, wobei das Auflageschnittstellenkissen mit einem Befestigungsmaterial ausgestaltet ist, das sich entlang einer Länge sowohl der ersten Wand als auch der zweiten Wand erstreckt.

7. Auflageschnittstellenkissen nach Anspruch 1, wobei die konkave Vertiefung dazu ausgestaltet ist, ein Probandenschnittstellenkissen so darin zu positionieren, dass eine Körperunterseite des Probandenschnittstellenkissens in flächigem Kontakt mit der konkaven Vertiefung steht.

8. Auflageschnittstellenkissen nach Anspruch 1, wobei die konkave Vertiefung dazu ausgestaltet ist, mindestens ein abgewinkeltes Positionierungskissen so darin zu positionieren, dass eine Basis eines Körpers des abgewinkelten Positionierungskissens in flächigem Kontakt mit der planaren ersten Seite der Basis des Auflageschnittstellenkissens steht.

9. Auflageschnittstellenkissen nach Anspruch 8, wobei sowohl das mindestens eine abgewinkelte Positionierungskissen als auch das Probandenschnittstellenkissen unter Verwendung eines Kopplungsgurts und/oder von Befestigungselementen und Aufnahmen dazwischen gekoppelt sind.

10. Probandenpositionierungssatz, umfassend:
das Auflageschnittstellenkissen (300) nach einem der vorhergehenden Ansprüche;
mindestens ein abgewinkeltes Positionierungskissen (600), das dazu ausgestaltet ist, in der konkaven Vertiefung des Auflageschnittstellenkissens positioniert zu werden; und
ein Probandenschnittstellenkissen (400, 500), das dazu ausgestaltet ist, in der konkaven Vertiefung des Auflageschnittstellenkissens positioniert zu werden, und
ferner dazu ausgestaltet ist, in flächigem Kontakt mit einer Innenfläche (610) des mindestens einen abgewinkelten Positionierungskissens positioniert zu werden.

11. Probandenpositionierungssatz nach Anspruch 10, wobei in einer ersten Ausgestaltung:
eine Körperunterseite des Probandenschnittstellenkissens dazu ausgestaltet ist, in flächigem Kontakt mit der Innenfläche eines ersten abgewinkelten Positionierungskissens des mindestens einen abgewinkelten Positionierungskissens positioniert zu werden; und
eine Basis eines Körpers des ersten abgewinkelten Positionierungskissens dazu ausgestaltet ist, in flächigem Kontakt mit der Innenfläche eines zweiten abgewinkelten Positionierungskissens des mindestens einen abgewinkelten Positionierungskissens positioniert zu werden und/oder in flächigem Kontakt mit der konkaven Vertiefung des Auflageschnittstellenkissens positioniert zu werden.

12. Probandenpositionierungssatz nach Anspruch 11, wobei, wenn sich die Basis des Körpers des ersten abgewinkelten Positionierungskissens in flächigem Kontakt mit der Innenfläche des zweiten abgewinkelten Positionierungskissens befindet, sich die Basis des Körpers des zweiten abgewinkelten Positionierungskissens in flächigem Kontakt mit der konkaven Vertiefung des Auflageschnittstellenkissens befindet.

13. Probandenpositionierungssatz nach Anspruch 11, wobei das Probandenschnittstellenkissen mit einem Befestigungsmaterial an der Körperunterseite des Probandenschnittstellenkissens ausgestaltet ist und sowohl die konkave Vertiefung des Auflageschnittstellenkissens als auch ein Inneres des mindestens einen abgewinkelten Positionierungskissens so mit einem Aufnahmematerial ausgestaltet sind, das komplementär zu dem Befestigungsmaterial des Probandenschnittstellenkissens ist, dass das Probandenschnittstellenkissen mit der konkaven Vertiefung des Auflageschnittstellenkissens und/oder dem Inneren des mindestens einen abgewinkelten Positionierungskissens gekoppelt wird, indem das Befestigungsmaterial mit dem Aufnahmematerial in Eingriff gebracht wird.

14. Probandenpositionierungssatz nach Anspruch 13, wobei das mindestens eine abgewinkelte Positionierungskissen ferner so mit dem Befestigungsmaterial an der Basis des Körpers des abgewinkelten Positionierungskissens ausgestaltet ist, dass;
das abgewinkelte Positionierungskissen mit der konkaven Vertiefung des Auflageschnittstellenkissens gekoppelt wird, indem das Befestigungsmaterial des abgewinkelten Positionierungskissens mit dem Aufnahmematerial des Auflageschnittstellenkissens in Eingriff gebracht wird, und;
das abgewinkelte Positionierungskissen mit dem zweiten abgewinkelten Positionierungskissen, das die gleiche Ausgestaltung wie das abgewinkelte Positionierungskissen aufweist, gekoppelt wird, indem das Befestigungsmaterial des abgewinkelten Positionierungskissens mit dem Aufnahmematerial des zweiten abgewinkelten Positionierungskissens in Eingriff gebracht wird.

15. Probandenpositionierungssatz nach Anspruch 10, wobei in einer zweiten Ausgestaltung ein erstes Ende des mindestens einen abgewinkelten Positionierungskissens in die gleiche Richtung wie ein erstes Ende des Auflageschnittstellenkissens weisend positioniert wird, um einen positiven Winkel zu erzeugen, und wobei in einer dritten Ausgestaltung das erste Ende des mindestens einen abgewinkelten Positionierungskissens in die gleiche Richtung wie ein zweites Ende des Auflageschnittstellenkissen gegenüber dem ersten Ende weist, um einen negativen Winkel zu erzeugen.

## Revendications

1. Pavé d'interface de berceau (300) destiné à être utilisé avec un système d'imagerie, le pavé d'interface de berceau comprenant :
une base (304) avec un premier côté plan (310) et un second côté incurvé (312), opposé au premier côté plan ;
une première paroi (306) positionnée sur une première extrémité de la base et une seconde paroi (308) positionnée sur une seconde extrémité de la base, la première paroi, la seconde paroi et le premier côté plan de la base formant un évidement concave du pavé d'interface de berceau ;
au moins une fente (326, 328, 330, 332) s'étendant à travers chacune de la première paroi et de la seconde paroi ;
au moins un point de fixation (342, 352) monté sur chacune de la première paroi et de la seconde paroi et s'étendant vers la base ; et
une poignée (344, 346) positionnée sur une face supérieure de chacune de la première paroi et de la seconde paroi, les poignées étant configurées pour fournir un élément du pavé d'interface de berceau permettant à un patient de le saisir avec ses mains pendant l'imagerie afin d'aider à éloigner les mains, les bras et les épaules du patient d'une région à imager.

2. Pavé d'interface de berceau selon la revendication 1, l'au moins une fente comprenant une première fente et une seconde fente, la première fente étant positionnée au-dessus de la seconde fente.

3. Pavé d'interface de berceau selon la revendication 2, la seconde fente étant parallèle à un bord supérieur de la première paroi et la première fente étant inclinée selon un angle non nul, par rapport au bord supérieur de la première paroi.

4. Pavé d'interface de berceau selon la revendication 2, comprenant en outre une sangle de positionnement s'étendant à travers chacune de la première fente sur la première paroi et la seconde paroi et/ou la sangle de positionnement s'étendant à travers chacune de la seconde fente sur la première paroi et la seconde paroi.

5. Pavé d'interface de berceau selon la revendication 1, chacun de l'au moins un point de fixation comprenant une extrémité de crochet configurée pour s'interfacer avec un rail d'une table, le point de fixation comprenant une section de montage (372), une extension incurvée (374) couplée à la section de montage (372), et une extrémité de crochet (376) couplée à l'extension incurvée (374).

6. Pavé d'interface de berceau selon la revendication 1, le pavé d'interface de berceau étant configuré avec un matériau de fixation qui s'étend le long d'une longueur de chacune de la première paroi et de la seconde paroi.

7. Pavé d'interface de berceau selon la revendication 1, l'évidement concave étant configuré pour avoir un pavé d'interface de sujet positionné à l'intérieur, de telle sorte qu'un dessous de corps du pavé d'interface de sujet est en contact à faces partagées avec l'évidement concave.

8. Pavé d'interface de berceau selon la revendication 1, l'évidement concave étant configuré pour avoir au moins un pavé de positionnement angulaire positionné à l'intérieur, de telle sorte qu'une base d'un corps du pavé de positionnement angulaire est en contact à faces partagées avec le premier côté plan de la base du pavé d'interface de berceau.

9. Pavé d'interface de berceau selon la revendication 8, chacun de l'au moins un pavé de positionnement angulaire et d'un pavé d'interface de sujet étant couplé en utilisant une sangle de couplage et/ou des attaches et des récepteurs entre eux.

10. Kit de positionnement de sujet, comprenant :
le pavé d'interface de berceau (300) selon l'une quelconque des revendications précédentes ;
au moins un pavé de positionnement angulaire (600) configuré pour être positionné dans l'évidement concave du pavé d'interface de berceau ; et
un pavé d'interface de sujet (400, 500) configuré pour être positionné dans l'évidement concave du pavé d'interface de berceau et configuré en outre pour être positionné en contact à faces partagées avec une surface intérieure (610) de l'au moins un pavé de positionnement angulaire.

11. Kit de positionnement de sujet selon la revendication 10, dans une première configuration :
un dessous de corps du pavé d'interface de sujet étant configuré pour être positionné en contact à faces partagées avec la surface intérieure d'un premier pavé de positionnement angulaire de l'au moins un pavé de positionnement angulaire ; et
une base d'un corps du premier pavé de positionnement angulaire étant configurée pour être positionnée en contact à faces partagées avec la surface intérieure d'un second pavé de positionnement angulaire de l'au moins un pavé de positionnement angulaire et/ou pour être positionnée en contact à faces partagées avec l'évidement concave du pavé d'interface de berceau.

12. Kit de positionnement de sujet selon la revendication 11, lorsque la base du corps du premier pavé de positionnement angulaire est en contact à faces partagées avec la surface intérieure du second pavé de positionnement angulaire, la base du corps du second pavé de positionnement angulaire étant en contact à faces partagées avec l'évidement concave du pavé d'interface de berceau.

13. Kit de positionnement de sujet selon la revendication 11, le pavé d'interface de sujet étant configuré avec un matériau de fixation sur le dessous de corps du pavé d'interface de sujet et chacun de l'évidement concave du pavé d'interface de berceau et d'un intérieur de l'au moins un pavé de positionnement angulaire étant configuré avec un matériau de réception qui est complémentaire au matériau de fixation du pavé d'interface de sujet, de telle sorte que le pavé d'interface de sujet est couplé à l'évidement concave du pavé d'interface de berceau et/ou à l'intérieur de l'au moins un pavé de positionnement angulaire par accouplement du matériau de fixation avec le matériau de réception.

14. Kit de positionnement de sujet selon la revendication 13, l'au moins un pavé de positionnement angulaire étant en outre configuré avec le matériau de fixation sur la base du corps du pavé de positionnement angulaire, de telle sorte que ;
le pavé de positionnement angulaire est couplé à l'évidement concave du pavé d'interface de berceau par accouplement du matériau de fixation du pavé de positionnement angulaire avec le matériau de réception du pavé d'interface de berceau et ;
le pavé de positionnement angulaire est couplé au second pavé de positionnement angulaire, présentant la même configuration que le pavé de positionnement angulaire, par accouplement du matériau de fixation du pavé de positionnement angulaire avec le matériau de réception du second pavé de positionnement angulaire.

15. Kit de positionnement de sujet selon la revendication 10, dans une deuxième configuration, une première extrémité de l'au moins un pavé de positionnement angulaire étant positionnée dans la même direction qu'une première extrémité du pavé d'interface de berceau pour créer un angle positif et, dans une troisième configuration, la première extrémité de l'au moins un pavé de positionnement angulaire dans la même direction qu'une seconde extrémité du pavé d'interface de berceau, opposée à la première extrémité, pour créer un angle négatif.
